# EUROPEAN PATENT APPLICATION

(11) **EP 4 563 142 A1**
(43) Date of publication of application: **04.06.2025**
(21) Application number: 23845896.2
(22) Date of filing: 09.03.2023
(51) Int. Cl.: A61K 9/127, A61K 31/7105, A61K 47/10, A61K 47/18, A61K 47/22, A61K 47/24, A61K 47/28, A61P 1/16

(54) **LIPID NANOPARTICLE AND PHARMACEUTICAL COMPOSITION**

(30) Priority: 29.07.2022 JP 2022121626
(71) Applicant: National University Corporation Hokkaido University, Hokkaido 060-0808 (JP)
(72) Inventor: HARASHIMA, Hideyoshi, Sapporo-shi, Hokkaido 060-0808 (JP); MAHMOUD, Abubakr Ahmed Younis, Sapporo-shi, Hokkaido 060-0808 (JP); SATO, Yusuke, Sapporo-shi, Hokkaido 060-0808 (JP)
(74) Representative: Bjerkén Hynell KB
(86) International application number: PCT/JP2023/009082
(87) International publication number: WO 2024/024156

(57) **Abstract**

The present invention relates to lipid nanoparticles capable of delivering a target substance to hepatic stellate cells. The lipid nanoparticles are for delivering a target substance to hepatic stellate cells and comprise a pH-sensitive cationic lipid including a hydrophilic portion and two hydrophobic portions, wherein an acid dissociation constant pKa of a lipid membrane constituting the lipid nanoparticles is greater than or equal to 6.7 and less than 8.2.

## Description

### Technical Field

The present invention relates to lipid nanoparticles for delivering a target substance to hepatic stellate cells, and a pharmaceutical composition for preventing or treating liver fibrosis.

### Background Art

Hepatic fibrosis is a chronic progressive disease manifested by excessive deposition of thick extracellular matrices (Non-Patent Literature (hereinafter, referred to as NPL) 1). Activated hepatic stellate cells (HSCs) play critical roles in hepatic fibrogenesis (NPL 2). Current treatment for hepatic fibrosis rely on low-molecular-weight inhibitors such as bismodegib and arsenic trioxide, which are poorly selective and toxic, as well as liver-transplantation that is not economical to many patients (NPL 3).

Genetic therapy targeting hepatic stellate cells is considered to be a promising innovative strategy for the treatment of hepatic fibrosis. However, gene therapy targeting hepatic stellate cells is limited by a number of challenges. The challenges include, for example, the difficulty of introducing nanomedicines into the thin hepatic sinusoids, the difficulty for the nanomedicines introduced into the hepatic sinusoids to diffuse outside the sinusoids, the overactivation of hepatic Kupffer cells, the physical barrier of thick interstitium that prevents nanomedicines from reaching hepatic stellate cells, the number of hepatic stellate cells in the liver being small, and hepatic stellate cells having difficulty to transfect (NPLs 4 and 5). Conventional delivery systems targeting hepatic stellate cells utilize ligands that have affinity for hepatic stellate cells, such as vitamin A, pPB peptide, and D-FNB peptide (NPLs 6 to 8).

On the other hand, in recent years, lipid nanoparticles including pH-sensitive cationic lipids capable of efficiently delivering siRNA to immune cells, cancer cells, and the like has been proposed (Patent Literatures (hereinafter, each referred to as PTL) 1 and 2.

### Citation List

### Patent Literature

PTL 1
   WO2018/230710
PTL 2
   Japanese Patent Application Laid-Open No. 2019-151589

### Non-Patent Literature

NPL 1
   Ramon Bataller and David A. Brenner, "Liver fibrosis", The Journal of Clinical Investigation, Vol. 115, pp. 209-218
NPL 2
   Dakota R. Kamm and Kyle S. McCommis, "Hepatic stellate cells in physiology and pathology", The Journal of Physiology, Vol. 600, pp. 1825-1837
NPL 3
   Zui Tan, et al., "Liver Fibrosis: Therapeutic Targets and Advances in Drug Therapy", Frontiers in Cell and Developmental Biology, Vol. 9, p. 730176
NPL 4
   Mahmoud A. Younis, Ikramy A. Khalil and Hideyoshi Harashima, "Gene Therapy for Hepatocellular Carcinoma: Highlighting the Journey from Theory to Clinical Applications", Vol. 3, p. 2000087
NPL 5
   Claus Kordes, et al., "Hepatic stellate cells: current state and open questions", Biological Chemistry, Vol. 402, p. 1021-1032
NPL 6
   Hirotaka Furuhashi, et al., "Vitamin A-coupled liposome system targeting free cholesterol accumulation in hepatic stellate cells offers a beneficial therapeutic strategy for liver fibrosis", Hepatology Reserach, Vol. 48, pp. 397-407
NPL 7
   Luying Huang, et al., "Highly Selective Targeting of Hepatic Stellate Cells for Liver Fibrosis Treatment Using a d-Enantiomeric Peptide Ligand of Fn14 Identified by Mirror-Image mRNA Display", Molecular Pharmaceutics, Vol. 14, pp. 1742-1753
NPL 8
   Jinfang Zhang, et al., "Liver-Targeted siRNA Lipid Nanoparticles Treat Hepatic Cirrhosis by Dual Antifibrotic and Anti-inflammatory Activities", ACS Nano, Vol. 14, pp. 6305-6322
NPL 9
   Mahmoud A. Younis, et al., "Clinical translation of nanomedicines: Challenges, opportunities, and keys", Advanced Drug Delivery Reviews, Vol. 181, p. 114083

### Summary of Invention

### Technical Problem

However, delivery systems utilizing the above ligands are not stable and not clinically applicable (NPL 9). Therefore, it is expected to develop a new delivery system targeting hepatic stellate cells.

An object of the present invention is to provide a lipid nanoparticle capable of delivering a target substance to a hepatic stellate cell. Another object of the present invention is to provide a pharmaceutical composition and a method for preventing or treating liver fibrosis.

### Solution to Problem

The present invention relates to the following lipid nanoparticle, pharmaceutical composition, and method of prophylaxis or treatment.
[1] A lipid nanoparticle for delivering a target substance to a hepatic stellate cell, the lipid nanoparticle including: a pH-sensitive cationic lipid including a hydrophilic portion and two hydrophobic portions, in which an acid dissociation constant pKa of a lipid membrane constituting the lipid nanoparticle is 6.7 or more and less than 8.2.
[2] The lipid nanoparticle according to [1], in which the hydrophilic portion includes a 5-7 membered non-aromatic heterocyclic group bonded to a carbonyl group.
[3] The lipid nanoparticle according to [2], in which: the two hydrophobic portions each independently includes an alkylene group having 4 to 12 carbon atoms, and an aliphatic group bonded to the alkylene group or a fatty acid ester group bonded to the alkylene group; the aliphatic group is a saturated or unsaturated aliphatic group having 8 to 18 carbon atoms and having 0 to 2 unsaturated bonds; and the fatty acid ester group is a linear or branched saturated or unsaturated fatty acid ester group having 10 to 24 carbon atoms and having 0 to 2 unsaturated bonds.
[4] The lipid nanoparticle according to [1], in which: the hydrophilic portion includes, at the end thereof, an ionizable tertiary amino group; and the two hydrophobic portions each independently include an alkylene group having 4 to 12 carbon atoms and a fatty acid ester group having 15 or more carbon atoms, the fatty acid ester group being bonded to the alkylene group.
[5] The lipid nanoparticle according to [3], in which:
   the pH-sensitive cationic lipid is represented by a general formula (1) below:

      (R¹)(R²)C(OH)-(CH₂)ₐ-(O-CO)-X¹ ... (1)
   in the general formula (1), a represents an integer of 3 to 5, and R¹ and R² each independently represent a group represented by a general formula (2) or (3) below:

      CH₃-(CH₂)_{q}-(CH=CH)ᵣ-(CH₂)ₛ-(CH=CH)t-(CH₂)ᵤ-(CO-O)_{c}-(CH₂)ᵥ- ... (2)

      in the general formula (2), q represents an integer of 1 to 9, r represents 0 or 1, s represents an integer of 1 to 3, t represents 0 or 1, u represents an integer of 1 to 8, c represents 0 or 1, and v represents an integer of 4 to 12, and

         (R³)(R⁴)C-(CO-O)-(CH₂)ᵥ- ... (3)
      in the general formula (3), R³ and R⁴ are each independently a C₃₋₁₁ alkyl group, and v is an integer of 4 to 12, and
   X¹ represents the 5-7 membered non-aromatic heterocyclic group that is bonded to (O-CO)- via a carbon atom, and a hydrogen atom on a ring of the 5-7 membered non-aromatic heterocyclic group is optionally substituted with one or two C₁₋₄ alkyl groups or C₂₋₄ alkenyl groups.
[6] The lipid nanoparticle according to [4], in which:
   the pH-sensitive cationic lipid is represented by a general formula (4) below:

      (R⁵)(R⁶)C(OH)-(CH₂)ₐ-X² ... (4)
   in the general formula (4), a represents an integer of 3 to 5, and R⁵ and R⁶ each independently represent a group represented by a general formula (5) below:

      CH₃-(CH₂)_{w}-(CH=CH)ₓ-(CH₂)_{y}-(CO-O)-(CH₂)ᵥ- ... (5)

      in the general formula (5), w represents an integer of 1 to 15, x represents 0 or 1, y represents an integer of 1 to 15, and v represents an integer of 4 to 12, and
   X² represents an ionizable tertiary amino group.
[7] The lipid nanoparticle according to [5], in which:
   the X¹ is a 1-pyrrolidinyl group, a 1-piperidinyl group, a 1-morpholinyl group, or a 1-piperazinyl group; and a hydrogen atom of the 1-pyrrolidinyl group, the 1-piperidinyl group, the 1-morpholinyl group or the 1-piperazinyl group is optionally substituted with one C₁₋₄ alkyl group.
[8] The lipid nanoparticle according to any one of [1] to [7], in which the acid dissociation constant pKa of the lipid membrane constituting the lipid nanoparticle is in a range of 6.7 to 7.4.
[9] The lipid nanoparticle according to any one of [1] to [8], in which a number average particle size of the lipid nanoparticle measured by dynamic light scattering is 100 nm or less.
[10] The lipid nanoparticle according to any one of [1] to [9], in which a ratio of the pH-sensitive cationic lipid to total lipids constituting the lipid nanoparticle is 20 mol% or more.
[11] The lipid nanoparticle according to [10], further including:
   20 to 30 mol% of a phospholipid containing a phosphoethanolamine group with respect to the total lipids constituting the lipid nanoparticle; 20 to 30 mol% of cholesterol with respect to the total lipids constituting the lipid nanoparticle; and 3 to 5 mol% of a polyalkylene glycol modified lipid with respect to the total lipids constituting the lipid nanoparticle.
[12] The lipid nanoparticle according to any one of [1] to [11], in which the target substance is RNA.
[13] The lipid nanoparticle according to [12], in which: the target substance is mRNA; and a nitrogen/phosphate ratio of the lipid nanoparticle is in a range of 6 to 14.
[14] The lipid nanoparticle according to [12], in which: the target substance is siRNA; and a nitrogen/phosphate ratio of the lipid nanoparticle is in a range of 4 to 8.
[15] The lipid nanoparticle according to any one of [1] to [14], in which the lipid nanoparticle does not include a ligand for binding to the hepatic stellate cell.
[16] A pharmaceutical composition for preventing or treating liver fibrosis, the pharmaceutical composition including:
   a lipid nanoparticle having delivery selectivity to a hepatic stellate cell; a first substance that is encapsulated in the lipid nanoparticle and suppresses an expression of a SMO gene or a SMO protein or inhibits the SMO protein; and a second substance that is encapsulated in the lipid nanoparticle and suppresses an expression of a TGFβ1 gene or a TGFβ1 protein or inhibits the TGFβ1 protein.
[17] The pharmaceutical composition according to [16], in which the lipid nanoparticle is the lipid nanoparticle according to any one of [1] to [15].
[18] The pharmaceutical composition according to [16] or [17], in which:
   the first substance is siRNA, miRNA, shRNA, an antisense oligonucleotide, or a component of CRISPR-Cas each capable of suppressing the expression of the SMO gene; and the second substance is siRNA, miRNA, shRNA, an antisense oligonucleotide, or a component of CRISPR-Cas each capable of suppressing the expression of the TGFβ1 gene.
[19] A method for preventing or treating liver fibrosis, the method including: administering the pharmaceutical composition according to any one of [16] to [18].

### Advantageous Effects of Invention

The present invention can provide a lipid nanoparticle capable of delivering a target substance to a hepatic stellate cell. The present invention can also provide a pharmaceutical composition and a method for preventing or treating liver fibrosis.

### Brief Description of Drawings

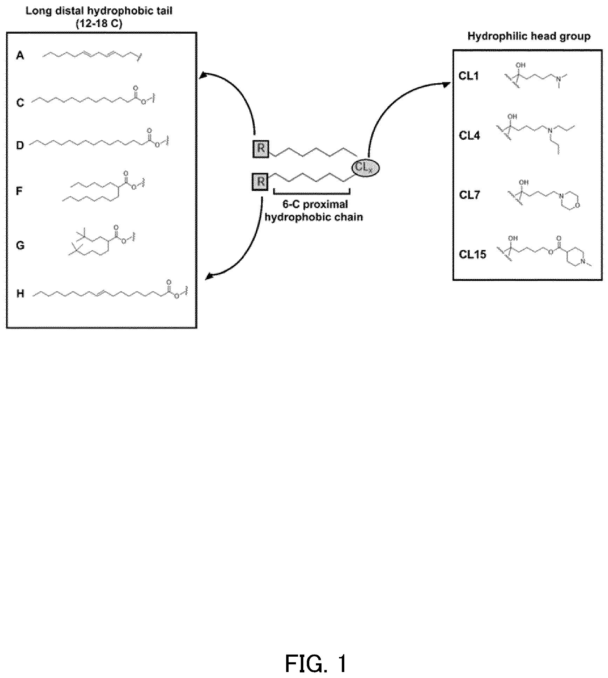

FIG. 1 illustrates a configuration of a pH-sensitive cationic lipid;

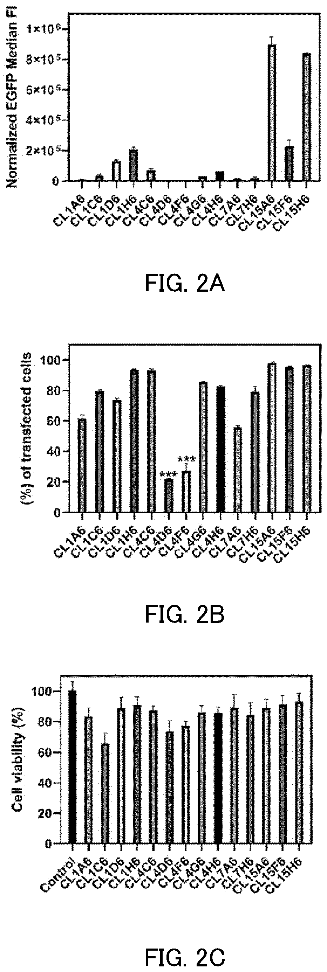

FIG. 2A is a graph showing the relationship between the type of pH-sensitive cationic lipid and mRNA delivery efficiency, FIG. 2B is a graph showing the relationship between the type of pH-sensitive cationic lipid and transfection efficiency, FIG. 2C is a graph showing the relationship between the type of pH-sensitive cationic lipid and cell viability;

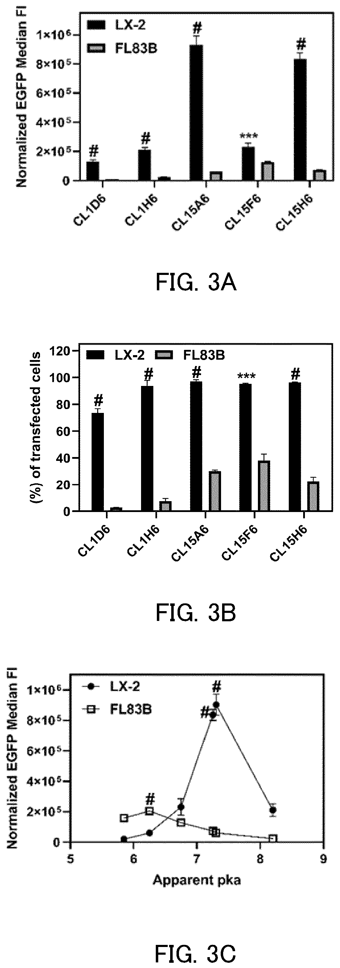

FIG. 3A is a graph showing the relationship between the type of pH-sensitive cationic lipid and mRNA delivery efficiency, FIG. 3B is a graph showing the relationship between the type of pH-sensitive cationic lipid and transfection efficiency, FIG. 3C is a graph showing the relationship between the pKa of pH-sensitive cationic lipids and the mRNA delivery efficiency;

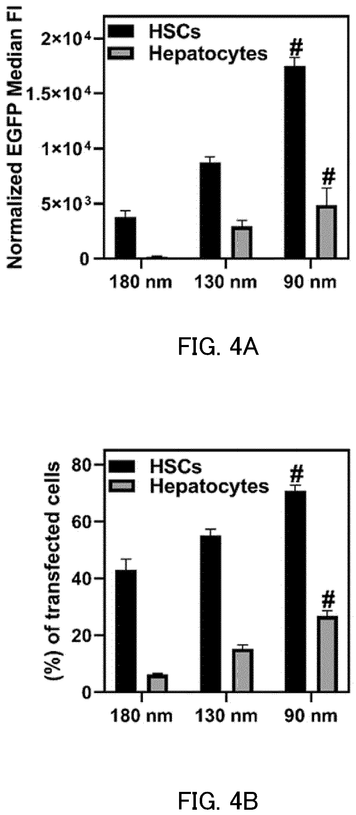

FIG. 4A is a graph showing the relationship between the particle size of lipid nanoparticles prepared by using CL15A6 and mRNA delivery efficiency, FIG. 4B is a graph showing the relationship between the particle size of the lipid nanoparticles prepared by using CL15A6 and the transfection efficiency;

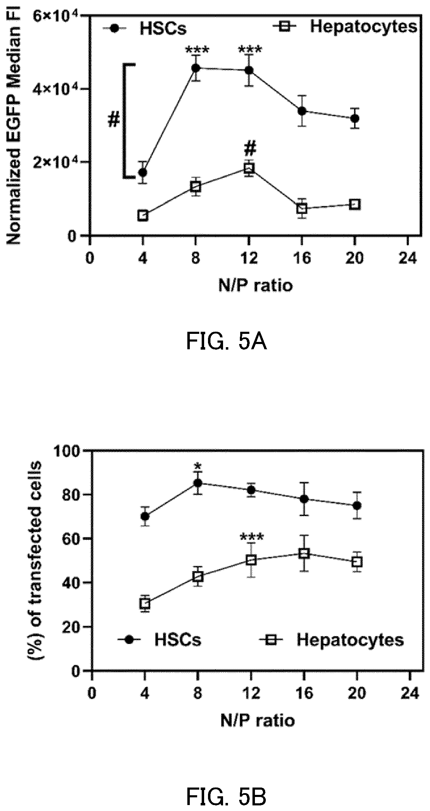

FIG. 5A is a graph showing the relationship between the N/P ratio of the lipid nanoparticles prepared by using CL15A6 and the mRNA delivery efficiency, and FIG. 5B is a graph showing the relationship between the N/P ratio of the lipid nanoparticles prepared by using CL15A6 and the transfection efficiency;

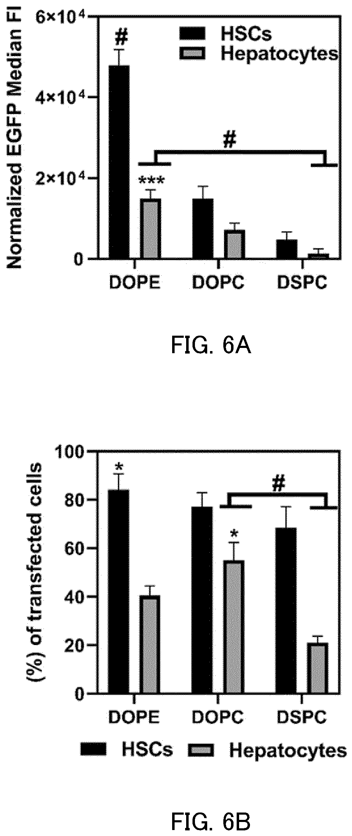

FIG. 6A is a graph showing the relationship between the type of helper phospholipid in the lipid nanoparticles prepared by using CL 15A6 and the mRNA delivery efficiency, and FIG. 6B is a graph showing the relationship between the type of helper phospholipid in the lipid nanoparticles prepared by using CL15A6 and the transfection efficiency;

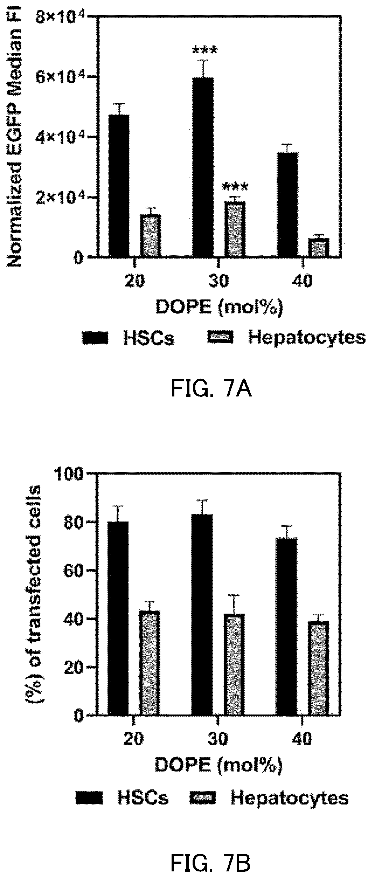

FIG. 7A is a graph showing the relationship between the proportion of DOPE in the lipid nanoparticles prepared by using CL15A6 and the mRNA delivery efficiency, and FIG. 7B is a graph showing the relationship between the proportion of DOPE in the lipid nanoparticles prepared by using CL15A6 and the transfection efficiency;

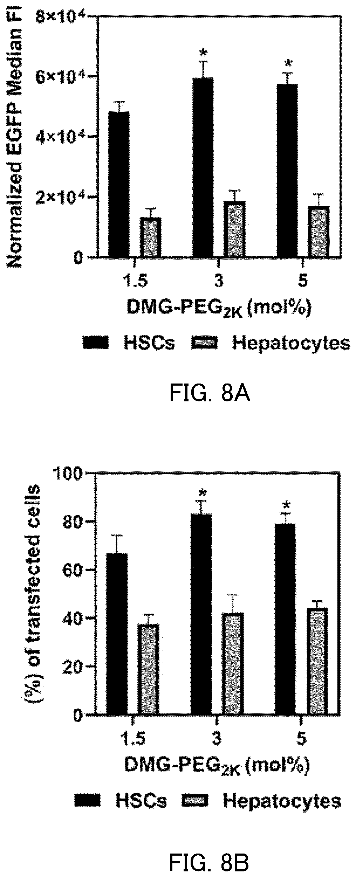

FIG. 8A is a graph showing the relationship between the proportion of DMG-PEG2K in the lipid nanoparticles prepared by using CL 15A6 and the mRNA delivery efficiency, and FIG. 8B is a graph showing the relationship between the proportion of the DMG-PEG2K in the lipid nanoparticles prepared by using CL15A6 and the transfection efficiency;

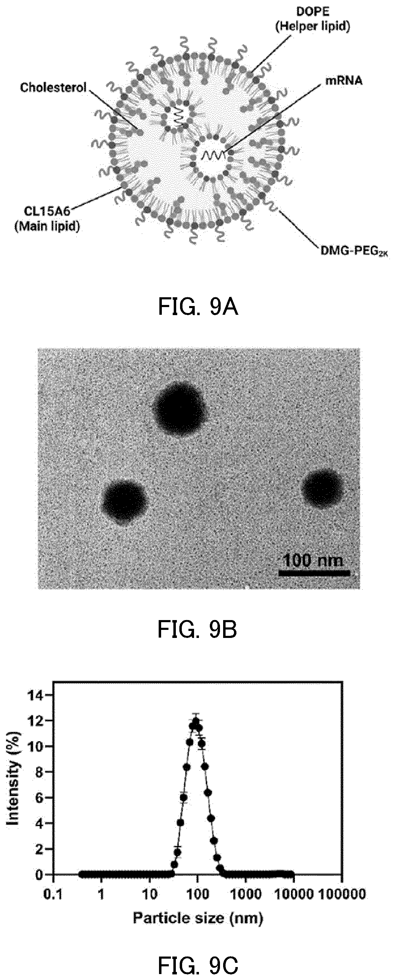

FIG. 9A is a schematic diagram showing the configuration of the lipid nanoparticle, FIG. 9B is a transmission electron microscope image of the lipid nanoparticle, FIG. 9C is a graph showing the particle size distribution of the lipid nanoparticles;

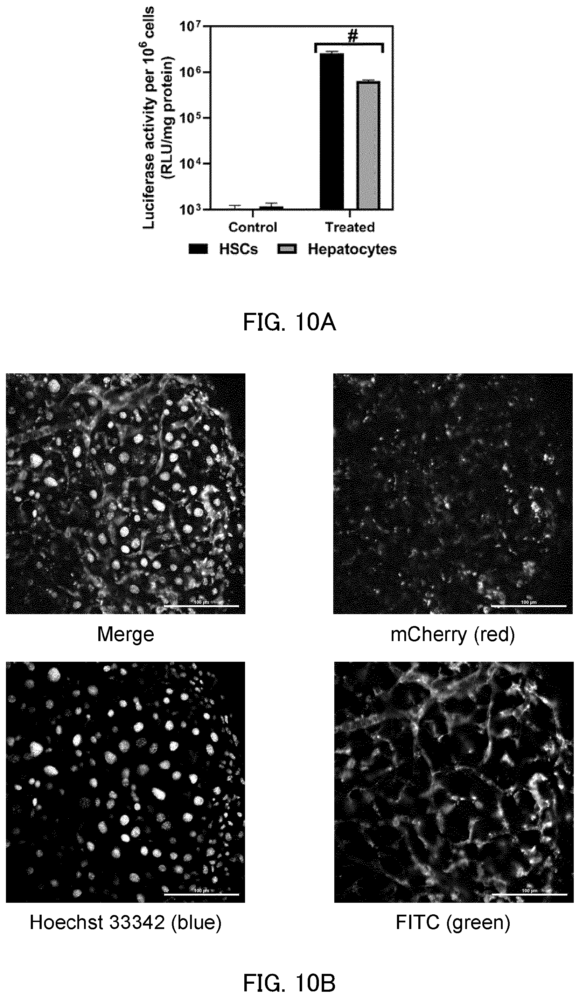

FIG. 10A is a graph showing the luciferase activity of HSCs of a liver after the administration of lipid nanoparticles encapsulating FLuc mRNA, and FIG. 10B is a fluorescent observation image of a liver tissue section after the administration of lipid nanoparticles encapsulating mCherry mRNA;

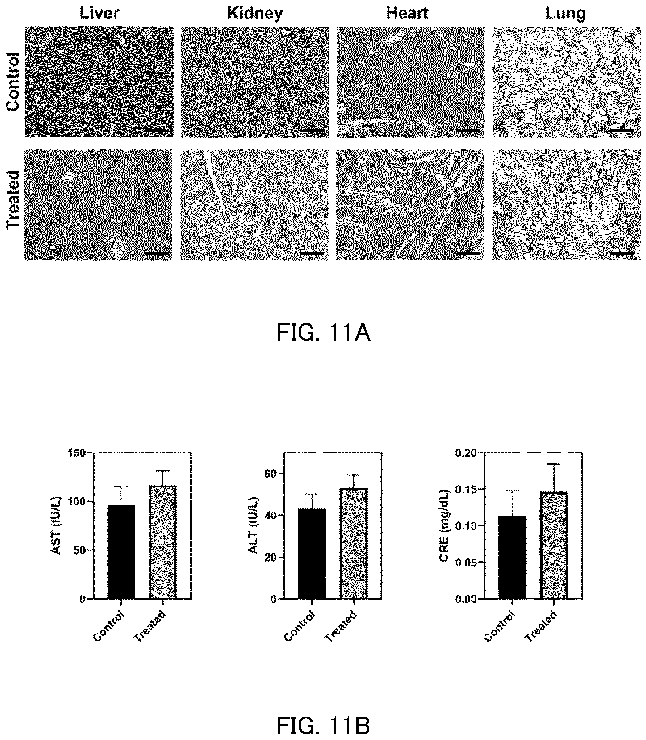

FIG. 11A illustrates photographs showing the histology of the major organs of a mouse after the administration of the lipid nanoparticles prepared by using CL15A6, and FIG. 11B is a graph showing the measured liver or kidney toxicity markers in the serum of the mouse after the administration of the lipid nanoparticles prepared by using CL15A6;

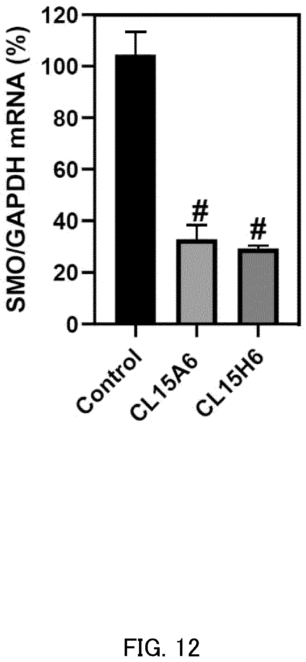

FIG. 12 is a graph showing the proportion of the amount of SMO mRNA when the lipid nanoparticles prepared by using CL15A6 were administered, the proportion of the amount of SMO mRNA when lipid nanoparticles prepared by using CL15H6 were administered, and the proportion of the amount of SMO mRNA when no lipid nanoparticles were administered (control);

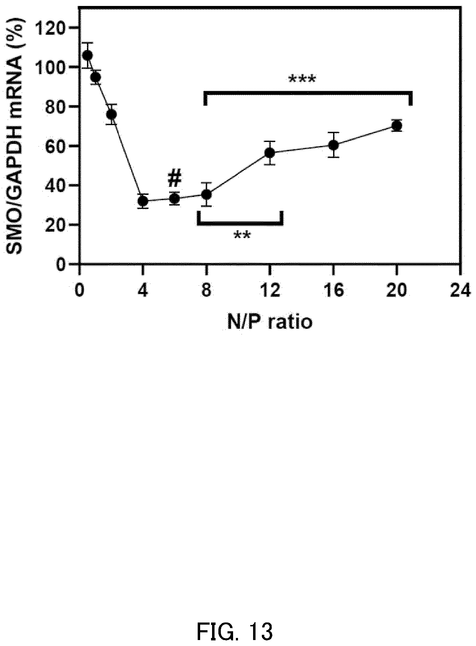

FIG. 13 is a graph showing the relationship between the N/P ratio of the lipid nanoparticles prepared by using CL15H6 and the proportion of the amount of SMO mRNA;

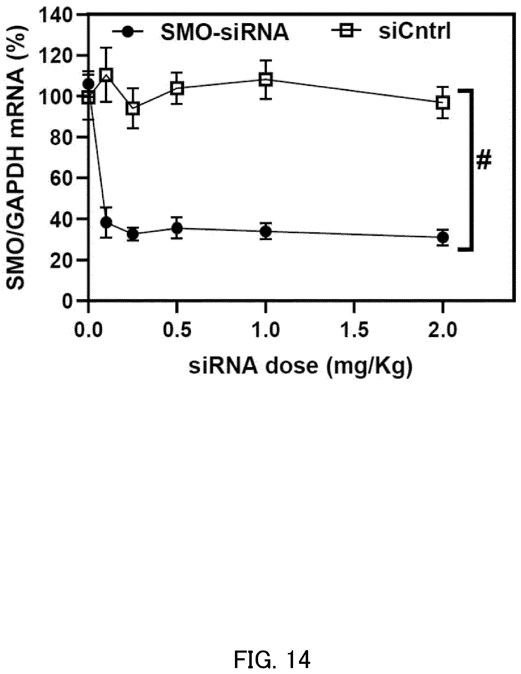

FIG. 14 is a graph showing the dose-response curve of the lipid nanoparticles prepared by using CL15H6;

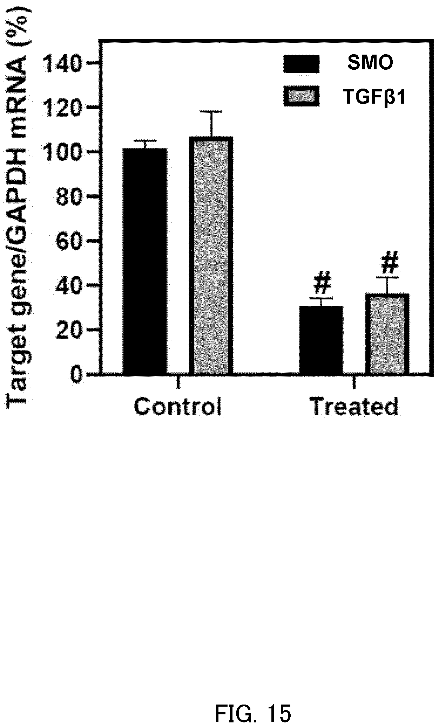

FIG. 15 is a graph showing the proportion of the amount of mRNA of the target gene when lipid nanoparticles prepared by using CL15H6 were administered, and the proportion of the amount of mRNA of the target gene when lipid nanoparticles were not administered (control);

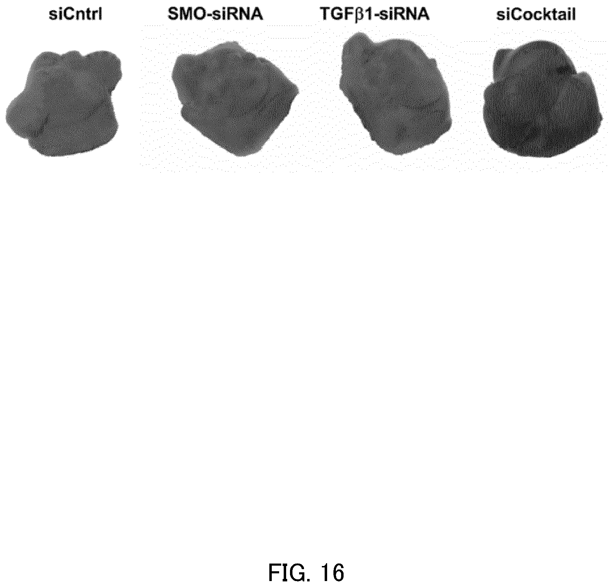

FIG. 16 illustrates photographs of the livers of mice administered with lipid nanoparticles containing siRNA;

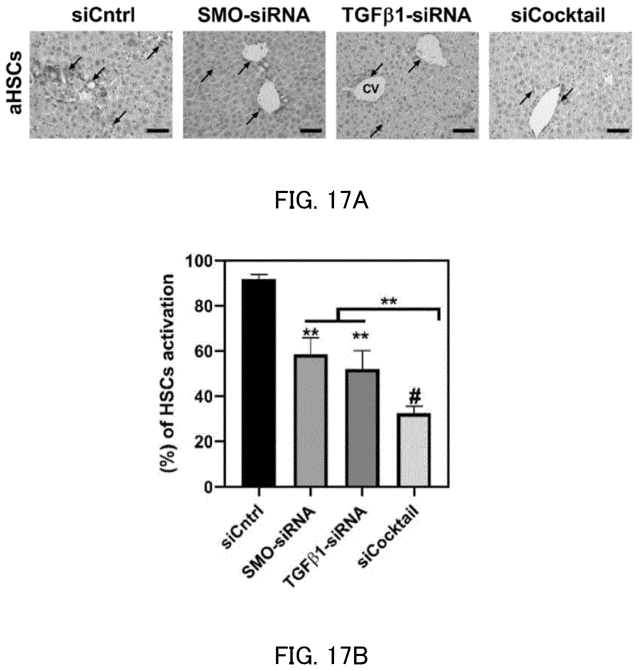

FIG. 17A illustrates stained images of anti-α-SMA antibodies in the sections of the livers of the mice administered with the lipid nanoparticles encapsulating siRNA, and FIG. 17B is a graph showing the proportions of aHSC in the livers of the mice administered with lipid nanoparticles encapsulating siRNA;

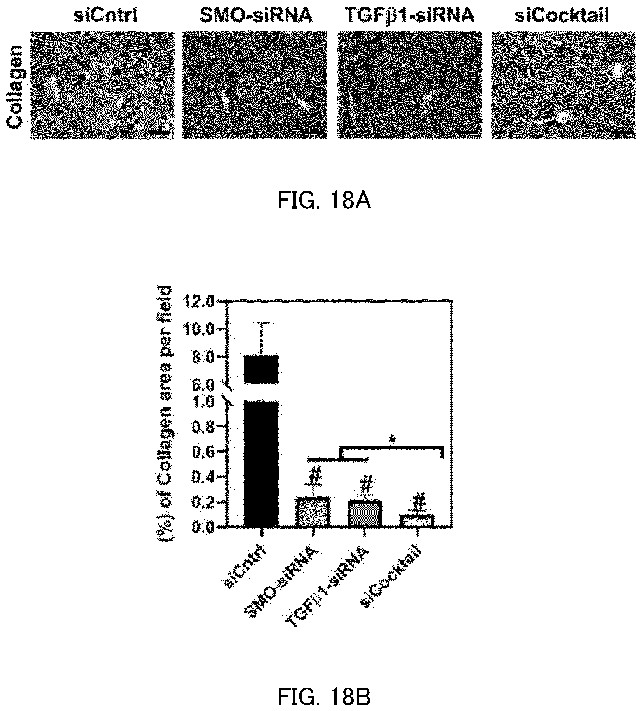

FIG. 18A illustrates Masson's Trichrome stained images of the liver sections from mice administered with lipid nanoparticles encapsulating siRNA, and FIG. 18B is a graph showing the proportions of collagen areas within fields of view in the livers of mice administered with the lipid nanoparticles encapsulating siRNA;

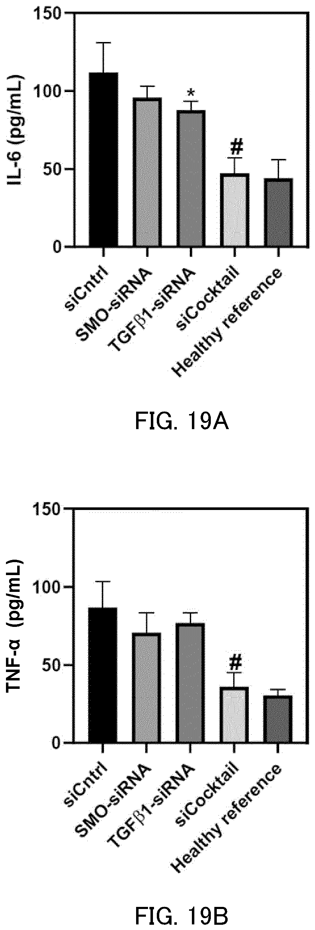

FIG. 19A is a graph showing the concentrations of IL-6 in the sera of the mice administered with the lipid nanoparticles encapsulating siRNA, and FIG. 19B is a graph showing the concentrations of TNF-α in the sera of the mice administered with the lipid nanoparticles encapsulating siRNA;

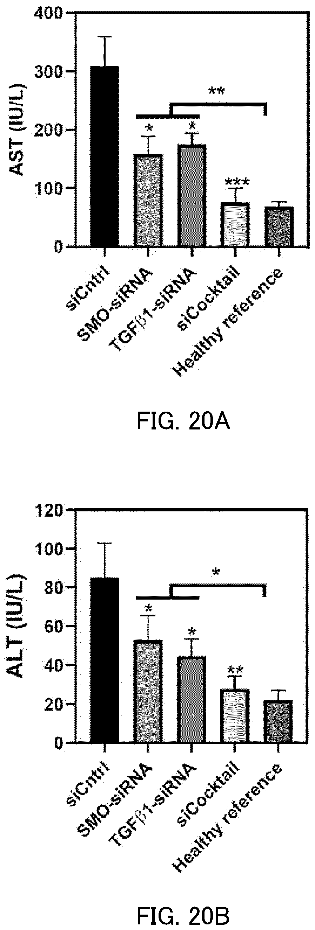

FIG. 20A is a graph showing the concentrations of AST in the sera of the mice administered with the lipid nanoparticles encapsulating siRNA, and FIG. 20B is a graph showing the concentrations of ALT in the sera of the mice administered with the lipid nanoparticles encapsulating siRNA; and

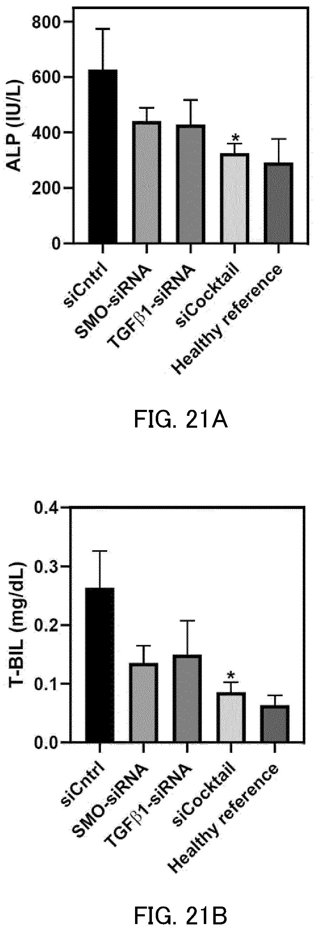

FIG. 21A is a graph showing the concentrations of ALP in the sera of the mice administered with the lipid nanoparticles encapsulating siRNA, and FIG. 21B is a graph showing the concentrations of T-BIL in the sera of the mice administered with the lipid nanoparticles encapsulating siRNA.

### Description of Embodiments

Hereinafter, embodiments of the present invention will be described in detail.

Herein, a numerical range expressed using "to" or "-" means a range that includes the numerical values written immediately before and after "to" or "-" as the lower limit and upper limit. In numerical ranges described stepwise herein, the upper or lower limit value described in a certain numerical range may be replaced with the upper or lower limit value of another numerical range in the ranges described stepwise.

### 1. Lipid nanoparticle

A lipid nanoparticle according to the present invention is a lipid nanoparticle for delivering a target substance to hepatic stellate cells, and contains a predetermined pH-sensitive cationic lipid including a hydrophilic portion and two hydrophobic portions. The acid dissociation constant pKa of a lipid membrane constituting the lipid nanoparticle is 6.7 or more and less than 8.2.

A lipid nanoparticle according to an embodiment of the present invention includes a pH-sensitive cationic lipid in which the hydrophilic portion includes a 5-7 membered non-aromatic heterocyclic group bonded to a carbonyl group of the pH-sensitive cationic lipid. In this pH-sensitive cationic lipid, it is preferred that the two hydrophobic portions each independently includes an alkylene group having 4 to 12 carbon atoms, and an aliphatic group bonded to the alkylene group or fatty acid ester group bonded to the alkylene group. It is preferred that the aliphatic group is a saturated or unsaturated aliphatic group having 8 to 18 carbon atoms and having 0 to 2 unsaturated bonds, and the fatty acid ester group is a linear or branched saturated or unsaturated fatty acid ester group having 10 to 24 carbon atoms and having 0 to 2 unsaturated bonds.

The carbon number of the aliphatic groups is not particularly limited as long as it is within a range of 8 to 18, but is preferably within a range of 10 to 16, more preferably within a range of 10 to 14, and particularly preferably 12. The aliphatic group is preferably an unsaturated aliphatic group, and particularly preferably an aliphatic group having two unsaturated bonds. Furthermore, the aliphatic group is preferably a linear aliphatic group.

The carbon number of the fatty acid ester groups is not particularly limited as long as it is within a range of 10 to 24, but is preferably within a range of 12 to 22, more preferably within a range of 14 to 20, and particularly preferably 16 to 18. The fatty acid ester group may be linear or branched, and may be saturated or unsaturated. When the fatty acid ester group is a branched fatty acid ester group, the fatty acid ester group is preferably a saturated fatty acid ester group. On the other hand, when the fatty acid ester group is a linear fatty acid ester group, the fatty acid ester group is preferably an unsaturated fatty acid ester group, and particularly preferably a fatty acid ester group having one unsaturated bond.

The two hydrophobic portions may be a combination of one hydrophobic portion containing an aliphatic group and one hydrophobic portion containing a fatty acid ester group. However, it is preferred that the two hydrophobic portions are a combination of two hydrophobic portions both containing the aliphatic group, or a combination of two hydrophobic portions both containing the fatty acid ester group. The two hydrophobic portions may be the same or different from each other, but are preferably the same.

For example, the lipid nanoparticle according to one embodiment of the present invention contains, as the pH-sensitive cationic lipid, a pH-sensitive cationic lipid represented by the following general formula (1).

(R¹)(R²)C(OH)-(CH₂)ₐ-(O-CO)-X¹ ... (1)

In the general formula (1), a represents an integer of 3 to 5, preferably 4.

In the general formula (1), R¹ and R² each independently represent a group represented by general formula (2) or general formula (3) below.

CH₃-(CH₂)_{q}-(CH=CH)ᵣ-(CH₂)ₛ-(CH=CH)ₜ-(CH₂)ᵤ-(CO-O)_{c}-(CH₂)ᵥ- ... (2)

(R³)(R⁴)C-(CO-O)-(CH₂)ᵥ- ... (3)

In the general formula (2), q represents an integer of 1 to 9, r represents 0 or 1, s represents an integer of 1 to 3, t represents 0 or 1, u represents an integer of 1 to 8, c represents 0 or 1, and v represents an integer of 4 to 12.

When c is 0 in the general formula (2), 1 + q + 2r + s + 2t + u is in the range of 8 to 18, preferably in the range of 10 to 16, more preferably in the range of 10 to 14, and particularly preferably 12. Further, r + t is preferably 1 or 2, and particularly preferably 2.

When c is 1 in the general formula (2), 1 + q + 2r + s + 2t + u + c is in the range of 10 to 24, preferably in the range of 12 to 22, more preferably in the range of 14 to 20, and particularly preferably in the range of 16 to 18. Further, r + t is preferably 1 or 2, and particularly preferably 1.

In the general formula (3), R³ and R⁴ are each independently a C₃₋₁₁ alkyl group, and v is an integer of 4 to 12.

In the general formula (3), the total number of carbon atoms of R³ and R⁴ is not particularly limited as long as it is within a range of 8 to 22, but is preferably within a range of 10 to 20, more preferably within a range of 12 to 18, and particularly preferably 14 to 16.

In the general formula (1), X¹ is a 5-7 membered non-aromatic heterocyclic group. The heterocyclic group is bonded to (O-CO)- via a carbon atom. A hydrogen atom on the ring of the heterocyclic group may be substituted with one or two C₁₋₄ alkyl groups (alkyl group having 1 to 4 carbon atoms) or C₂₋₄ alkenyl groups (alkenyl having 2 to 4 carbon atoms). Examples of the heteroatom included in the heterocyclic group include nitrogen, oxygen, and sulfur atoms. The heterocyclic group may include one heteroatom or two or more of the same or different heteroatoms. The heterocycle of the heterocyclic group may include one or more double bonds, but is not an aromatic ring. The heterocycle is preferably a saturated heterocycle. Among the substituents that substitute one or two hydrogen atoms in heterocyclic group, examples of the C₁₋₄ alkyl group includes methyl, ethyl, n-propyl, n-butyl, isopropyl, isopropyl, isobutyl and tert-butyl groups. Examples of the C₂₋₄ alkenyl group include ethenyl (vinyl), propenyl, and butenyl groups.

For example, X¹ is 1-pyrrolidinyl group, 1-piperidinyl group, 1-morpholinyl group, or 1-piperazinyl group. A hydrogen atom of the 1-pyrrolidinyl group, 1-piperidinyl group, 1-morpholinyl group or 1-piperazinyl group may be substituted with one C₁₋₄ alkyl group.

Among the compounds represented by the general formula (1), preferred compounds having a group represented by the general formula (2) include a compound in which R¹ and R² are the same, a is 4, q is 4, r is 1, s is 1, t is 1, u is 2, c is 0, v is 6, and X¹ is a 1-methylpiperidinyl group. This is the compound ((14z,17z)-5-hydroxy-5-((9z,12z)-octadeca-9,12-dien-1-yl)tricosa-14,17-dien-1-yl 1-methylpiperidine-4-carboxylate), which is referred to as "CL15A6" in the Examples described below.

Among the compounds represented by the general formula (1), examples of another preferred compound having a group represented by the general formula (2) include a compound in which R¹ and R² are the same, a is 4, q is 7, r is 1, s is 0, t is 0, u is 7, c is 1, v is 6, and X¹ is a 1-methylpiperidinyl group. This is the compound (7-hydroxy-7-(4-((1-methylpiperidine-4-carbonyl)oxy)butyl)tridecane-1,13-diyl dioleate), which is referred to as "CL15H6" in the Examples described below.

Among the compounds of the general formula (1), preferred compounds having a group represented by the general formula (3) include a compound in which R³ is a hexyl group, R⁴ is an octyl group, v is 6, and X¹ is a 1-methylpiperidinyl group. This is the compound (7-hydroxy-7-(4-((1-methylpiperidine-4-carbonyl)oxy)butyl)tridecane-1,13-diyl bis(2-hexyldecanoate)), which is referred to as "CL15F6" in the Examples described below.

In a lipid nanoparticle according to another embodiment of the present invention, the hydrophilic portion includes at the end thereof an ionizable tertiary amino group, and the two hydrophobic portions each independently include an alkylene group having 4 to 12 carbon atoms and a fatty acid ester group having 15 or more carbon atoms-the fatty acid ester group is bonded to the alkylene group.

The number of carbon atoms of the fatty acid ester group is not particularly limited as long as it is 15 or more, but is preferably 16 or more. The carbon number of the fatty acid ester group is preferably 24 or less, more preferably 22 or less, particularly preferably 20 or less, and most preferably 18 or less. The fatty acid ester group may be linear or branched, but is preferably a linear fatty acid ester group. The fatty acid ester group may be either saturated or unsaturated.

The two hydrophobic portions may be the same or different from each other, but are preferably the same.

A lipid nanoparticle according to another embodiment of the present invention includes, as pH-sensitive cationic lipid, a pH-sensitive cationic lipid represented by the following general formula (4).

(R⁵)(R⁶)C(OH)-(CH₂)ₐ-X² ... (4)

In the general formula (4), a represents an integer of 3 to 5, and is preferably 4.

In the general formula (4), R⁵ and R⁶ are each independently a group represented by the following general formula (5).

CH₃-(CH₂)_{w}-(CH=CH)ₓ-(CH₂)_{y}-(CO-O)-(CH₂)ᵥ- ... (5)

In the general formula (5), w represents an integer of 1 to 15, x represents 0 or 1, y represents an integer of 1 to 15, and v represents an integer of 4 to 12.

In the general formula (5), 1 + w + 2x + y + 1 is 15 or more, preferably 16 or more. Further, 1 + w + 2x + y + 1 is preferably 24 or less, more preferably 22 or less, particularly preferably 20 or less, and most preferably 18 or less.

In the general formula (4), X² is an ionizable tertiary amino group. For example, X² is a dimethylamino group.

Examples of preferred compounds among the compounds of the general formula (4) include a compound in which R⁵ and R⁶ are the same, a is 4, w is 7, x is 0, y is 7, v is 6 and X² is a dimethylamino group. This is the compound (7-(4-(dimethylamino)butyl)-7-hydroxytridecane-1,13-diyl dipalmitate), which is referred to in Examples below as "CL1D6."

Another example of a preferred compound among the compounds of the general formula (4) is a compound in which R⁵ and R⁶ are the same, a is 4, w is 7, x is 1, y is 7, v is 6, and X² is a dimethylamino group. This is the compound (7-(4-(dimethylamino)butyl)-7-hydroxytridecane-1,13-diyl dioleate), which is referred to in Examples below as "CL1H6."

The pH-sensitive cationic lipids including the above compounds can be easily prepared by a person skilled in the art. For example, a person skilled in the art can easily produce any compound included in the general formulas (1) and (4) by appropriately selecting a material compound, a reagent, a reaction condition, and the like, and referring to the production methods described in PTL 1 or 2 or NPL 10 described below. All of the disclosures of PTL 1, PTL 2 and NPL 10 are incorporated herein by reference.

The ratio of the pH-sensitive cationic lipid to the total lipids constituting the lipid nanoparticle according to the present invention is not particularly limited, but is preferably 20 mol% or more. For example, the ratio of the pH-sensitive cationic lipid is preferably in the range of 20 to 60 mol%, particularly preferably in the range of 35 to 60 mol%.

The lipid constituting the lipid membrane of the lipid nanoparticles according to the present invention may be only the pH-sensitive cationic lipid described above, but is preferably a combination of the above-described pH-sensitive cationic lipid with one or more other lipids. The type of lipid used in combination with the pH-sensitive cationic lipid described above is not particularly limited, and for example, a lipid used in forming liposomes can be used. Examples of such lipids include phospholipids, glycolipids, sterols, saturated or unsaturated fatty acid esters, saturated or unsaturated fatty acids, and the like.

Examples of the phospholipids and derivatives thereof include phosphatidylethanolamine, phosphatidylcholine, phosphatidylserine, phosphatidylinositol, phosphatidylglycerol, cardiolipin, sphingomyelin, ceramide phosphorylethanolamine, ceramide phosphorylglycerol, ceramide phosphorylglycerol phosphate, plasmalogen, phosphatidic acid, and the like. These may be used singly or in combination of two or more. The fatty acid residue in the phospholipids is not particularly limited, and may be, for example, a saturated or unsaturated fatty acid residue having 12 to 20 carbon atoms. Examples of such fatty acid residues include acyl groups derived from fatty acids such as lauric acid, myristic acid, palmitic acid, stearic acid, oleic acid, linoleic acid, and the like. Phospholipids derived from natural products such as egg yolk lecithin and soybean lecithin can also be used.

More specific examples of the phospholipids include 1,2-dioleoyl-sn-glycero-3-phosphoethanolamine (DOPE), 1,2-stearoyl-sn-glycero-3-phosphoethanolamine (DSPE), 1,2-dipalmitoyl-sn-glycero-3-phosphocholine (DPPC), and 1-palmitoyl-2-oleoyl-sn-glycero-3-phosphocholine (POPC). The lipid nanoparticles according to the present invention preferably contains a phospholipid, and more preferably contains a phospholipid having a phosphoethanolamine group, such as DOPE or DSPE.

Examples of the glycolipids include glyceroglycolipids such as sulfoxyribosylglyceride, diglycosyldiglyceride, digalactosyldiglyceride, galactosyldiglyceride, and glycosyldiglyceride; and sphingoglycolipids such as galactosylcerebroside, lactosylcerebroside, and ganglioside.

Examples of the sterols include sterols derived from animals, such as cholesterol, cholesterol succinate, lanosterol, dihydrolanosterol, desmosterol and dihydrocholesterol; sterols derived from plants, such as stigmasterol, sitosterol, campesterol and brassicasterol (phytosterols); sterols derived from microorganisms, such as zymosterol and ergosterol. The lipid nanoparticles according to the present invention preferably contains a sterol, and more preferably contains a cholesterol. By incorporating a sterol such as cholesterol into the lipid membrane constituting the lipid nanoparticles, the stability of the lipid nanoparticles can be increased.

Examples of the saturated or unsaturated fatty acids include saturated or unsaturated fatty acids having 12 to 20 carbon atoms, such as palmitic acid, oleic acid, stearic acid, arachidonic acid, myristic acid, and the like. Examples of the saturated or unsaturated fatty acid esters include glycerol fatty acid esters in which one or two hydroxyl groups of glycerol are ester-bonded to a fatty acid. Examples of the fatty acid residues in glycerol fatty acid esters include acyl groups derived from saturated or unsaturated fatty acids having 12 to 20 carbon atoms, such as palmitic acid, oleic acid, stearic acid, arachidonic acid, myristic acid, and the like. For example, the fatty acid ester is dimyristoylglycerol (DMG) or distearoylglycerol (DSG).

A lipid nanoparticle of the present invention may be surface-modified. For example, the surface of the lipid nanoparticle may be modified with an oligosaccharide compound having three or more sugar units to facilitate nuclear translocation of lipid nanoparticle. The type of the oligosaccharide compound having three or more sugar units is not particularly limited, but is, for example, an oligosaccharide compound having about 3 to 10 sugar units bonded thereto, and preferably an oligosaccharide compound having about 3 to 6 sugar units bonded thereto.

Specific examples of the oligosaccharide compounds include trisaccharide compounds such as cellotriose (β-D-glucopyranosyl-(1→4)-β-D-glucopyranosyl-(1→4)-D-glucose), chacotriose (α-L-rhamnopyranosyl-(1→2)-[α-L-rhamnopyranosyl-(1→4)]-D-glucose), gentianose (β-D-fructofuranosyl-β-D-glucopyranosyl-(1→6)-α-D-glucopyranoside), isomaltotriose (α-D-glucopyranosyl-(1→6)-α-D-glucopyranosyl-(1→6)-D-glucose), isopanose (α-D-glucopyranosyl-(1→4)-[α-D-glucopyranosyl-(1→6)]-D-glucose), maltotriose (α-D-glucopyranosyl-(1→4)-α-D-glucopyranosyl-(1→4)-D-glucose), manninotriose (α-D-galactopyranosyl-(1→6)-α-D-galactopyranosyl-(1→6)-D-glucose), melezitose (α-D-glucopyranosyl-(1→3)-β-D-fructofuranosyl = α-D-glucopyranoside), panose (α-D-glucopyranosyl-(1→6)-α-D-glucopyranosyl-(1→4)-D-glucose), planteose (α-D-galactopyranosyl-(1-6) -β-D-fructofuranosyl = α-D-glucopyranoside), raffinose (β-D-fructofuranosyl = α-D-galactopyranosyl-(1→6)-α-D-glucopyranoside), solatriose (α-L-rhamnopyranosyl-(1→2)-[β-D-glucopyranosyl-(1→3)]-D-galactose), umbelliferose (β-D-fructofuranosyl = α-D-galactopyranosyl-(1→2)-α-D-galactopyranoside). Specific examples of the oligosaccharide compounds also include tetrasaccharide compounds such as lycotetraose (β-D-glucopyranosyl-(1→2)-[β-D-xylopyranosyl-(1→3)]-β-D-glucopyranosyl-(1→4)-β-D-galactose), maltotetraose (α-D-glucopyranosyl-(1→4)-α-D-glucopyranosyl-(1→4)-α-D-glucopyranosyl-(1→4)-D-glucose), and stachyose (β-D-fructofuranosyl=α-D-galactopyranosyl-(1→6)-α-D-galactopyranosyl-(1→6)-α-D-glucopyranoside). Specific examples of oligosaccharide compounds also include pentasaccharide compounds such as maltopentaose (α-D-glucopyranosyl-(1→4)-α-D-glucopyranosyl-(1→4)-α-D-glucopyranosyl-(1→4)-α-D-glucopyranosyl-(1→4)-D-glucose) and verbascose (β-D-fructofuranosyl = α-D-galactopyranosyl-(1→6)-α-D-galactopyranosyl-(1→6)-α-D-galactopyranosyl-(1→6)-α-D-glucopyranoside). Specific examples of oligosaccharide compounds also include hexasaccharide compounds such as maltohexaose (α-D-glucopyranosyl-(1→4)-α-D-glucopyranosyl-(1→4)-α-D-glucopyranosyl-(1→4)-α-D-glucopyranosyl-(1→4)-α-D-glucopyranosyl-(1→4)-D-glucopyranosyl-(1→4)-D-glucose).

The method for modifying the surface of the lipid nanoparticles with an oligosaccharide compound is not particularly limited. For example, liposomes obtained by modifying the surface of lipid nanoparticles with a monosaccharide such as galactose or mannose (PTL 3) are known, and therefore the surface modification method described in this publication may be employed. This method uses a monosaccharide compound to be bonded to a polyalkylene glycolated lipid to modify the surface of lipid nanoparticles, and therefore is preferable as the surface of the lipid nanoparticles can be simultaneously modified with a polyalkylene glycol.
[PTL 3] WO2007/102481

By modifying the surface of lipid nanoparticles with a hydrophilic polymer such as a polyalkylene glycol, the stability, such as blood retention, of the lipid nanoparticles can be increased. This method is described, for example, in PTLs 4 to 7. The hydrophilic polymer is preferably a polyalkylene glycol. Examples of the polyalkylene glycol include polyethylene glycol (PEG), polypropylene glycol, polytetramethylene glycol and polyhexamethylene glycol. The molecular weight of the polyalkylene glycol is, for example, about 300 to 10000, preferably about 500 to 10000, and more preferably about 1000 to 5000.
[PTL 4] Japanese Patent Application Laid-Open No. H1-249717
[PTL 5] Japanese Patent Application Laid-Open No. H2-149512
[PTL 6] Japanese Patent Application Laid-Open No. H4-346918
[PTL 7] Japanese Patent Application Laid-Open No. 2004-10481

The modification of the surface of lipid nanoparticles with a polyalkylene glycol can be easily performed, for example, by using a polyalkylene glycol modified lipid as a constituent lipid of the lipid membrane. For example, when modification with polyethylene glycol is performed, stearylated polyethylene glycol (e.g., PEG 45 stearate (STR-PEG45) and the like) can be used. In addition, polyethylene glycol derivatives such as N-[carbonyl-methoxypolyethylene glycol-2000]-1,2-dipalmitoyl-sn-glycero-3-phosphoethanolamine, N-[carbonyl-methoxypolyethylene glycol-5000]-1,2-dipalmitoyl-sn-glycero-3-phosphoethanolamine, N-[carbonyl-methoxypolyethylene glycol-750]-1,2-distearoyl-sn-glycero-3-phosphoethanolamine, N-[carbonyl-methoxypolyethylene glycol-2000]-1,2-distearoyl-sn-glycero-3-phosphoethanolamine, and N-[carbonyl-methoxypolyethylene glycol-5000]-1,2-distearoyl-sn-glycero-3-phosphoethanolamine can also be used.

Further, by binding an oligosaccharide compound to an polyalkylene glycol, modification with the polyalkylene glycol and modification with the oligosaccharide compound can be performed simultaneously. However, the method of modifying lipid nanoparticles with a polyalkylene glycol or an oligosaccharide compound is not limited to the above method. For example, the surface of lipid nanoparticles can also be modified by using a lipidated compound such as a stearylated polyalkylene glycol or oligosaccharide compound as a constituent lipid of the lipid nanoparticles.

In the lipid nanoparticles of the present invention, for example, lipid derivatives for increasing blood retention, such as glycophorin, ganglioside GM1, phosphatidylinositol, ganglioside GM3, glucuronic acid derivatives, glutamic acid derivatives, and polyglycerol phospholipid derivatives, can also be used. In addition to the polyalkylene glycol, hydrophilic polymers for increasing the blood retention, such as dextran, pullulan, ficoll, polyvinyl alcohol, styrene-maleic anhydride alternating copolymer, divinyl ether-maleic anhydride alternating copolymer, amylose, amylopectin, chitosan, mannan, cyclodextrin, pectin, carrageenan, and the like can also be used.

The lipid nanoparticles of the present invention may include one or more substances selected from the group consisting of the following: membrane stabilizers such as sterols, glycerol, fatty acid esters of glycerol; antioxidants such as tocopherol, propyl gallate, ascorbyl palmitate, and butylated hydroxytoluene; charged substances; and membrane polypeptides. Examples of charged substances that impart a positive charge include saturated or unsaturated fatty amines such as stearylamine and oleylamine; saturated or unsaturated cationic synthetic lipids such as dioleoyltrimethylammonium propane; and cationic polymers. Examples of charged substances that impart a negative charge include dicetyl phosphate, cholesteryl hemisuccinate, phosphatidylserine, phosphatidylinositol, phosphatidic acid, and the like. Examples of the membrane polypeptides include, for example, peripheral membrane polypeptides, integral membrane polypeptides, and the like.

Further, the lipid nanoparticles of the present invention may be provided with any one or more functions such as, for example, a temperature-sensitive function, a membrane-permeation function, a genetic expression function, and a pH-sensitive function. By adding these functions as appropriate, for example, it is possible to improve the retention of the lipid nanoparticles in blood, or to enable the lipid nanoparticles to efficiently escape from endosomes after being taken up by target cells (hepatic stellate cells) by endocytosis and migrate into the nucleus.

Examples of temperature-sensitive lipid derivatives capable of imparting a temperature-sensitive function include dipalmitoylphosphatidylcholine, and the like. Examples of pH-sensitive lipid derivatives capable of imparting a pH-sensitive function include dioleoylphosphatidylethanolamine, and the like.

As shown in Examples below, lipid nanoparticles according to the present invention can be selectively taken up by hepatic stellate cells, which are target cells, even when the lipid nanoparticles do not include a ligand for binding to the hepatic stellate cells. That is, the lipid nanoparticles according to the present invention can selectively deliver an encapsulated target substance to hepatic stellate cells even in the absence of a ligand for binding to the hepatic stellate cells. Therefore, the lipid nanoparticles according to the present invention do not have to include a ligand for binding to hepatic stellate cells.

On the other hand, the lipid nanoparticles according to the present invention may be modified with a substance such as an antibody capable of specifically binding to a cell surface receptor or antigen. Such modifications can improve delivery efficiency to hepatic stellate cells. For example, a monoclonal antibody against a biological component specifically expressed in hepatic stellate cells may be placed on the surface of the lipid nanoparticle. By incorporating a lipid derivative capable of reacting with a mercapto group in a monoclonal antibody or a fragment thereof (e.g., Fab fragment, F(ab')₂ fragment, or Fab' fragment) as a constituent of the lipid membrane of the lipid nanoparticle, the monoclonal antibody can be bound to the surface of the lipid membrane of the lipid nanoparticle. Here, the lipid derivative is, for example, a lipid derivative having a maleinimide structure, such as poly(ethylene glycol)-α-distearoylphosphatidylethanolamine-ω-maleinimide, and α-[N-(1,2-distearoyl-sn-glycero-3-phosphoryl-ethyl)carbamyl)-ω-[3-[2-(2,5-dihydro-2,5-dioxo-1H-pyrrol-1-yl)ethanecarboxamido]propyl}-poly(oxy-1,2-ethanedyl).

The surface of the lipid nanoparticles of the present invention may be modified with a polypeptide including a plurality of consecutive arginine residues (hereinafter referred to as "polyarginine"). The polyarginine is preferably a polypeptide including 4 to 20 consecutive arginine residues, more preferably a polypeptide composed only of 4 to 20 consecutive arginine residues, particularly preferably octaarginine or the like. By modifying the surface of the lipid nanoparticle with a polyarginine such as octaarginine, the intracellular delivery efficiency of a target substance enclosed in the lipid nanoparticle can be improved. Modification of the surface of the lipid nanoparticles with polyarginine can be easily performed by using, for example, a lipid modified polyarginine such as stearylated octaarginine as a constituent lipid of the lipid nanoparticles.

When enclosing a nucleic acid such as mRNA or siRNA in the lipid nanoparticle of the present invention, a compound having a nucleic acid introduction function may be used as needed. Examples of a such compound include O,O'-N-didodecanoyl-N-(α-trimethylammonioacetyl)-diethanolamine chloride, O,O'-N-ditetradecanoyl-N-(α-trimethylammonioacetyl)-diethanolamine chloride, O,O'-N-dihexadecanoyl-N-(α-trimethylammonioacetyl)-diethanolamine chloride, O,O'-N-dioctadecenoyl-N-(α-trimethylammonioacetyl)-diethanolamine chloride, O,O',O"-tridecanoyl-N-(ω-trimethyl)ammoniodecanoyl)aminomethane bromide, N-[α-trimethylammonioacetyl]-didodecyl-D-glutamate, dimethyldioctadecylammonium bromide, 2,3-dioleyloxy-N-[2-(sperminecarboxamido)ethyl)-N,N-dimethyl-1-propaneammonium trifluoroacetate, 1,2-dimyristyloxypropyl-3-dimethyl-hydroxyethylammonium bromide, 3-β-[N-(N',N'-dimethylaminoethane)carbamoyl]cholesterol, and the like. Such a compound having the nucleic acid introduction function may be located at any position in the lipid membrane of the lipid nanoparticle, or may fill the inside of the lipid nanoparticle.

When the lipid nanoparticle according to the present invention includes one or more components, in addition to the above pH-sensitive cationic lipid, as a component of the lipid membrane, the proportions of the respective components are appropriately adjusted according to the object. For example, when the lipid nanoparticle according to the present invention includes the pH-sensitive cationic lipid as described above, a phospholipid containing a phosphoethanolamine group (e.g., DOPE), a sterol (e.g., cholesterol) and a polyalkylene glycol modified lipid (e.g., PEG modified lipid), the ratios of respective components to the total lipids constituting the lipid nanoparticle are preferably as follows: 20 to 57 mol% (preferably 35 to 57 mol%) of the pH-sensitive cationic lipid, 20 to 30 mol% of the phospholipid containing a phosphoethanolamine group (e.g., DOPE), 20 to 30 mol% of the sterol (e.g., cholesterol), and 3 to 5 mol% of the polyalkylene glycol modified lipid (e.g., PEG-modified lipid).

The lipid nanoparticle of the present invention is a lipid nanoparticle for delivering a target substance such as mRNA or siRNA to a hepatic stellate cells, characterized in that the target substance is enclosed in the lipid nanoparticle and the lipid nanoparticle includes the pH-sensitive cationic lipid as a lipid component. The target cell, namely a hepatic stellate cell, may be a cell in the body of an animal, or may be a cell cultured in vitro, such as a cultured cell or a primary cultured cell.

The type of target substance to be enclosed in the lipid nanoparticle of the present invention is not particularly limited. Examples of the target substance include nucleic acids such as messenger RNA (mRNA), small interfering RNA (siRNA), microRNA (miRNA), small hairpin RNA (shRNA), antisense oligonucleotide (ASO), components of CRISPR-Cas (guide RNA and Cas), and plasmids; active ingredients of any pharmaceutical agent such as antitumor agents, anti-inflammatory agents, antibacterial agents, and antiviral agents; sugars; peptides; small molecular weight compounds; metallic compounds, and the like.

The form of the lipid nanoparticle according to the present invention is not particularly limited. Exemplary forms of lipid nanoparticle dispersed in an aqueous solvent include single-membrane lipsomes, multilayered lipsomes, spherical micelles, string-shaped micelles, and irregular layered constructions. The lipid nanoparticle according to the present invention is preferably a single-membrane liposome or a multilayered liposome.

Enveloped nanostructures with added multifunctionality (MEND) are also known, and can be suitably used as a lipid nanoparticle of the present invention. For example, a MEND has been reported in which a complex of a nucleic acid such as plasmid DNA and a cationic polymer such as protamine is used as a core, and the core is enclosed inside a lipid envelop membrane in a liposomal form. It has also been reported that the MEND's lipid envelope membrane can be provided with peptides for regulating pH responsiveness and membrane permeability as needed, and the outer surface of the lipid envelope membrane can be modified with an alkylene glycol such as polyethylene glycol. Another MEND is also known which is designed to encapsulate condensed DNA and a cationic polymer inside the lipid envelope of the MEND, thereby enabling efficient gene expression.

The acid dissociation constant pKa of the lipid membrane of a lipid nanoparticle according to the present invention is not particularly limited as long as it is 6.7 or more and less than 8.2, but is more preferably 6.7 to 7.4, and particularly preferably about 7.0. The uptake of a lipid nanoparticle into a cell by endocytosis is affected by the pKa of the lipid that constitutes the lipid nanoparticle. The pKa of the lipid that is easily taken up by endocytosis varies depending on the type of cell. Therefore, it is preferable to adjust the acid dissociation constant pKa of the lipid membrane by, for example, selecting the type of substituent so that the pKa of the lipid is in a range that allows the lipid nanoparticle to be easily taken into target cells. In the present invention, by adjusting the apparent acid dissociation constant pKa of the lipid membrane to 6.7 or more and less than 8.2, the lipid nanoparticles are more likely to be selectively taken up by hepatic stellate cells (see Examples). The apparent acid dissociation constant pKa of the lipid membrane of a lipid nanoparticle is measured in a buffer at 37°C using 6-p-toluidino-2-naphthalenesulfonic acid (TNS) (see Examples).

The average particle size of the lipid nanoparticles according to the present invention is not particularly limited, but is preferably 100 nm or less. An average particle size of 100 nm or less promotes extravasation of the lipid nanoparticles and permeation of the lipid nanoparticles in a microenvironment rich in fibrotic liver stroma, making hepatic stellate cells more accessible to the lipid nanoparticles (see Example). The lower limit of the average particle diameter is not particularly limited, either. The average particle size of the lipid nanoparticles is, for example, equal to or greater than 10 nm, equal to or greater than 20 nm, or equal to or greater than 60 nm. Herein, the average particle size of lipid nanoparticles means the number average particle size measured by dynamic light scattering (DLS). The dynamic light scattering may be performed by a conventional method using a commercially available DLS device. The average particle size of the lipid nanoparticles may be adjusted by, for example, using a membrane filter having a predetermined pore size or the like to perform extrusion filtration under high pressure. In addition, the average particle size of lipid nanoparticles can also be easily adjusted by preparing lipid nanoparticles using a microchannel device (iLiNP device) described in NPL 11 described below. Specifically, as described in PTL 8, when the microchannel device is used to prepare lipid nanoparticles, a flow rate ratio of an aqueous phase to an organic phase, a buffer system, a lipid/nucleic acid ratio (N/P ratio), a proportion of a pH-sensitive cationic lipid, a proportion of a polyalkylene glycol modified lipid, and the like can be adjusted
[PTL 8] Japanese Patent Application Laid-Open No. 2021-172597

The polydispersity index (PDI) of the lipid nanoparticles according to the present invention is not particularly limited, but is, for example, about 0.05 to 0.1, preferably about 0.06 to 0.08, and more preferably about 0.07. The zeta potential is not particularly limited, but is preferably within a range of -16 to +5mV, more preferably within a range of -8 to +5mV, and particularly preferably in the vicinity of 0mV.

When a lipid nanoparticle according to the present invention includes a nucleic-acid acid such as RNA, the nitrogen/phosphate (N/P) ratio, which is the molar ratio of nitrogen atoms derived from the pH-sensitive cationic lipid to phosphoric acid derived from the nucleic acid, is not particularly limited. For example, when the lipid nanoparticle encapsulates mRNA, the N/P ratio is preferably in the range of 6 to 14, and particularly preferably in the range of 8 to 12. When the N/P ratio is within the above range, the endosomal escape capability of the lipid nanoparticle is improved, and transfection efficiency is improved. When the lipid nanoparticle encapsulates siRNA, the N/P ratio is preferably in the range of 4 to 8. When the N/P ratio is within the above range, the expression of target gene can be efficiently suppressed.

The state of the lipid nanoparticles is not particularly limited, but may be, for example, a state in which the lipid nanoparticles are dispersed in an aqueous solvent (for example, water, saline, phosphate buffered saline, or the like), or a state in which the aqueous dispersion is lyophilized.

The method for producing the lipid nanoparticles according to the present invention is not particularly limited, and any method available to a person skilled in the art can be employed. For example, lipid nanoparticles according to the present invention can be produced by dissolving all lipid components in an organic solvent such as chloroform, performing drying under reduced pressure using an evaporator and spray-drying using a spray-dryer to form a lipid membrane, and subsequently, adding an aqueous solvent to the dried mixture, and emulsifying the mixture using an emulsifier such as a homogenizer, an ultrasonic emulsifier, a high-pressure jet emulsifier, or the like. The lipid nanoparticles according to the present invention can also be produced by a method well known as a method for producing liposomes, for example, a reverse phase evaporating method or an alcoholic diluting method. Furthermore, a microchannel device can also be used to produce the lipid nanoparticles according to the present invention (see Examples).

The type of the aqueous solvent (dispersion medium) is not particularly limited. Examples of the aqueous solvent include phosphate buffer solutions, citrate buffer solutions, buffer solutions such as phosphate buffered saline, saline, culture media for cell cultures, and the like. These aqueous solvents can stably disperse lipid nanoparticles, and at least one of the following sugars and polyhydric alcohols (aqueous solutions) may be further added. Examples of the sugars include monosaccharides such as glucose, galactose, mannose, fructose, inositol, ribose, xylose, and the like, disaccharides such as lactose, sucrose, cellobiose, trehalose, maltose, trisaccharides such as raffinose and melezinose, polysaccharides such as cyclodextrin, sugar alcohols such as erythritol, xylitol, sorbitol, mannitol, maltitol, and the like. Examples of the polyhydric alcohols include polyhydric alcohols such as glycerol, diglycerin, polyglycerol, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol. In order to stably store lipid nanoparticles dispersed in an aqueous solvent for a long period of time, it is desirable to eliminate the electrolyte in the aqueous solvent as much as possible from the viewpoint of physical stability such as aggregation inhibition. From the viewpoint of chemical stability of lipid, it is desirable to set the pH of an aqueous solvent from weakly acidic to near neutral (pH3.0 to about 8.0) and remove dissolved oxygen by nitrogen bubbling or the like.

For lyophilizing or spray drying the resulting aqueous dispersion of the lipid nanoparticle, the stability can be improved by using, for example, at least one of the following sugars: monosaccharides such as glucose, galactose, mannose, fructose, inositol, ribose, and xylose; disaccharides such as lactose, sucrose, cellobiose, trehalose, and maltose; trisaccharides such as raffinose and melezinose; polysaccharides such as cyclodextrin; and sugar alcohols such as erythritol, xylitol, sorbitol, mannitol, and maltitol (aqueous solutions). In addition, for freezing the above-described aqueous dispersion, stability can be improved by, for example, using, for example, at least one of the above-described sugars and polyhydric alcohols such as glycerol, diglycerin, polyglycerol, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol.

By using the lipid nanoparticle according to the present invention, a target substance can be selectively delivered to a hepatic stellate cell. The delivery method can be performed in vivo in mammals, including humans, or in vitro using cells harvested from a living body. The lipid nanoparticle according to the present invention can also deliver a target substance to a hepatic stellate cell of a liver of hepatic fibrosis. The lipid nanoparticles according to the present invention can perform the above-described function even when the lipid nanoparticle does not include a ligand for binding to a hepatic stellate cell. Therefore, the lipid nanoparticles according to the present invention can be used to realize a rapid, economical, scalable, and clinically applicable delivery system for hepatic stellate cells.

2. Pharmaceutical composition and method for preventing or treating liver fibrosis A pharmaceutical composition and a method according to the present invention are for preventing or treating liver fibrosis. The pharmaceutical composition and the method can suppress or inhibit the Smoothened (SMO) gene or SMO protein in hepatic stellate cells, and can also suppress or inhibit the transforming growth factor-β1 (TGFβ1) gene or TGFβ1 protein. In this way, by suppressing or inhibiting both SMO and TGFβ1, the activity of hepatic stellate cells is suppressed, and liver fibrosis is suppressed and/or improved.

Liver fibrosis develops in response to damage to a liver, causing damage to hepatocytes and activation of Kupffer cells. Damaged hepatocytes release hedgehog ligands (Hh), which activate hepatic stellate cells' SMO signaling pathway and activate the hepatic stellate cells. The activated Kupffer cells release TGFβ1. The TGFβ1 activates the TGFβ1 signaling pathway of hepatic stellate cells and further activates the hepatic stellate cells. Therefore, by knocking down a SMO gene and a TGFβ1 gene at the same time, it is expected that hepatic stellate cells are inactivated and quiescent. Actually, knockdown of the SMO gene and the TGFβ1 gene at the same time can suppress and improve liver fibrosis, as shown in the Examples below.

More specifically, the pharmaceutical composition according to the present invention is for preventing or treating liver fibrosis, and the pharmaceutical composition includes lipid nanoparticles having delivery selectivity to hepatic stellate cells and a first substance and a second substance both encapsulated in the lipid nanoparticles. Here, the first substance is a substance that suppresses the expression of the SMO gene or SMO protein or inhibits the SMO protein. Similarly, the second substance is a substance that suppresses the expression of the TGFβ1 gene or TGFβ1 protein or inhibits the TGFβ1 protein.

In addition, the method according to the present invention is a method for preventing or treating liver fibrosis, and includes the step of administering the pharmaceutical composition according to the present invention.

The lipid nanoparticle is not particularly limited as long as the lipid nanoparticle has delivery selectivity to hepatic stellate cells, and may be, for example, the lipid nanoparticle according to the present invention. Other delivery vehicles with delivery selectivity to hepatic stellate cells may also be used in place of lipid nanoparticle.

The first substance is a substance that suppresses the expression of the SMO gene or SMO protein or inhibits the SMO protein as described above. Examples of the substance that suppresses the expression of the SMO gene or SMO protein include siRNA, miRNA, shRNA, antisense oligonucleotides, components of CRISPR-Cas (guide RNA and Cas), and the like. Examples of the substance that inhibits the SMO protein include antibodies, aptamers, and the like.

The second substance is a substance that suppresses the expression of the TGFβ1 gene or TGFβ1 protein or inhibits the TGFβ1 protein, as described above. Examples of the substance that suppresses the expression of the TGFβ1 gene or TGFβ1 protein include siRNA, miRNA, shRNA, antisense oligonucleotides, components of CRISPR-Cas (guide RNA and Cas), and the like. Examples of the substance that inhibits the TGFβ1 protein include antibodies and aptamers, and the like.

The siRNA is a double-stranded RNA capable of suppressing the expression of target gene (SMO gene or TGFβ1 gene) by RNAi action or DNA coding the double-stranded RNA. The siRNA is preferably double-stranded RNA having a continuous partial sequence in the base sequence of the RNA transcribed from the target gene, or DNA coding the double-stranded RNA. More specifically, the siRNA preferably has a sequence complementary to the continuous sequence of the number of bases required to suppress the expression of the target gene, which is of the CDS or UTR in the base sequence of the RNA transcribed from the target gene. The DNA coding the double-stranded RNA is, for example, a DNA having an inverted repeat sequence of the partial sequence of the target gene. By introducing such DNA having an inverted repeat sequence into a mammalian cell, the inverted repeat sequence of the partial sequence of the target gene can be expressed in the cell, and thus the expression of the target gene can be suppressed by the RNAi effect.

The miRNA can pair with the 3'UTR of mRNA to suppress the translation of a target gene (SMO gene or TGFβ1 gene). More specifically, the miRNA is transcribed as an RNA precursor having a hairpin-shaped structure, cleaved by a dsRNA cleaving enzyme having RNaseIII cleavage activity, and incorporated into a RISC or RISC-like protein complex to suppress the translation of mRNA. The miRNA may be any of primary miRNA (pri-miRNA), pre-miRNA, and mature miRNA. The miRNA preferably contains a continuous partial sequence in the 3'UTR of RNA transcribed from a target gene or a sequence complementary thereto. The length of the partial sequence is not particularly limited, and is preferably 7 bases or more, more preferably 8 bases or more, still preferably 9 bases or more, still more preferably 11 bases or more, particularly preferably 13 bases or more, particularly preferably 15 bases or more, and most preferably 17 bases or more. The upper limit of the length of the partial sequence is not particularly limited, and is preferably 50 bases or less, more preferably 40 bases or less, still more preferably 30 bases or less, particularly preferably 25 bases or less, and most preferably 23 bases or less. The length of the pri-miRNA is usually several hundred to several thousand bases, and the length of the pre-miRNA is usually 50 to 80 bases.

Tha shRNA is single-stranded RNA having an inverted repeat sequence in which a partial sequence in the base sequence of RNA transcribed from a target gene (SMO gene or TGFβ1 gene) and a complementary inverted sequence are juxtaposed via a sequence capable of forming a hairpin loop, or DNA coding the above RNA. The ShRNA can function similarly to the siRNA described above. Preferably, the shRNA has a sequence complementary to a continuous sequence of the number of bases required to suppress the expression of a target gene, which is of the CDS or UTR in the base sequence of RNA transcribed from a target gene. The length of the sequence capable of forming the hairpin loop is not particularly limited as long as the hairpin loop can be formed, but is preferably 0 to 300bp, more preferably 1 to 100bp, still more preferably 2 to 75bp, and particularly preferably 3 to 50bp. A restriction enzyme site may be present in this sequence.

The antisense oligonucleotide is an oligonucleotide having a sequence complementary to a continuous sequence in a CDS or UTR in an exon or intron of a target gene (SMO gene or TGFβ1 gene), or a continuous sequence in an expression control region of a target gene. Within a cell (preferably within the nucleus), an oligonucleotide contained in a target gene hybridizes with the above-described complementary antisense oligonucleotide, and the mRNA of the target gene containing a nucleotide chain is degraded by a nuclease (e.g., RNase H) specific to the resulting hybrid double strand, thereby suppressing transcription or translation of the target gene. The antisense oligonucleotide may be DNA or RNA, but is preferably DNA from the viewpoint of cleavage of mRNA by the specific nuclease. The antisense oligonucleotide is more preferably an antisense oligonucleotide having a sequence complementary to an oligonucleotide contained in CDS or UTR of the target gene, further preferably an antisense oligonucleotide having a sequence complementary to an oligonucleotide contained in UTR of the target gene, and particularly preferably an antisense oligonucleotide having a sequence complementary to an oligonucleotide contained in 3'UTR of the target gene. The length of the oligonucleotide is not particularly limited, and is preferably 10 bases or more, more preferably 11 bases or more, still more preferably 12 bases or more, still more preferably 13 bases or more, and particularly preferably 14 bases or more. The upper limit of the length of the oligonucleotide is not particularly limited, and is preferably 40 bases or less, more preferably 30 bases or less, still more preferably 25 bases or less, particularly preferably 20 bases or less, and most preferably 17 bases or less.

The components of CRISPR-Cas include guide RNA and Cas nuclease (e.g., Cas9). The guide RNA is RNA having a function of binding to a DNA cleavage enzyme (namely Cas nuclease), and guides the Cas nuclease to a target gene (SMO gene or TGFB1 gene). The guide RNA has at its 5' terminal a sequence complementary to the target DNA (the sequence contained in the target gene), and by binding to the target DNA via this complementary sequence, guides the Cas nuclease to the target gene. The Cas nuclease functions as a DNA endonuclease and can cleave DNA at the site where the target DNA is present to specifically reduce the expression of the target gene, for example. The target DNA is an oligonucleotide contained in the target gene (CDS or UTR in an exon or intron) or in the expression control region of the target gene. From the viewpoint of reliably reducing the expression of the target gene, the target DNA is preferably an oligonucleotide contained in the target gene (CDS or UTR in an exon) or in the expression control region of the target gene, more preferably an oligonucleotide contained in the target gene (CDS in an exon) or in the expression control region of the target gene, and even more preferably an oligonucleotide contained in the target gene (CDS in an exon). The partial sequence in the target gene serving as the target DNA is preferably 15 to 25 bases, more preferably 17 to 22 bases, still more preferably 18 to 21 bases, and particularly preferably 20 bases. By incorporating guide RNA specific to a target gene (SMO gene or TGFβ1 gene) or DNA coding the guide RNA and a nucleic acid coding a Cas nuclease or the Cas nuclease into the lipid nanoparticles, the expression of the target gene in hepatic stellate cells can be reduced.

The antibody that selectively binds to the target protein (SMO protein or TGFβ1 protein) may be either a polyclonal antibody or a monoclonal antibody as long as the antibody can specifically bind to the target protein. When the antibody is used for the purpose of administering to a human, it is preferable to use a human-type antibody or a humanized antibody in order to reduce immunogenicity. These human-type antibody and humanized antibody can be generated using a mammal, such as a transgenic mouse.

The aptamer that selectively bind to a target protein (SMO protein or TGFβ1 protein) is composed of single-stranded RNA or DNA, and binds to the target protein to inhibit the functions of the target protein due to the three-dimensional structure of the aptamer. Aptamers have high binding and specificity to target proteins, low immunogenicity, can be produced by chemical synthesis, and have high storage stability. The base length of the aptamer is not particularly limited as long as the aptamer can specifically bind to the target protein, but is preferably 15 to 60 bases, more preferably 20 to 50 bases, still more preferably 25 to 47 bases, and particularly preferably 26 to 45 bases. Aptamers that selectively bind to target proteins can be obtained, for example, by SELEX methods.

The pharmaceutical composition according to the present invention is administered to a subject suffering from or at risk of suffering from hepatic fibrosis, for example, one of mammals such as rodents including mice, rats, hamsters and guinea pigs, primates including humans, chimpanzees and rhesus monkeys, domestic animals including pigs, cows, goats, horses and sheep, and pets including dogs and cats. A preferred subject is a human.

The dosage form of the pharmaceutical composition according to the present invention is not particularly limited, but is preferably a parenteral preparation, and may be, for example, an injection, an infusion, or the like. In addition, the administration route of the pharmaceutical composition according to the present invention is not particularly limited. In the case of a parenteral preparation, for example, intravascular administration (preferably intravenous administration), intraperitoneal administration, intraintestinal administration, subcutaneous administration, local administration to a target site, and the like can be performed. In one preferred embodiment, the pharmaceutical composition according to the present invention is administered to a living body by intravenous or local administration. In addition, the dosage amount of the pharmaceutical composition according to the present invention is appropriately selected depending on the method of use and/or the age, sex, weight, and degree of liver fibrosis of the subject.

According to the pharmaceutical composition and method of the present invention, the activity of hepatic stellate cells can be suppressed by suppressing or inhibiting both SMO and TGFβ1 in hepatic stellate cells, and liver fibrosis can be suppressed and improved.

The present invention will be further described by the following Examples, but the present invention is not limited by these Examples.

### Examples

### [Example 1]

In Example 1, an experiment was carried out to deliver mRNA to hepatic stellate cells.

### 1. Preparation and characteristics evaluation of lipid nanoparticle encapsulating mRNA

### (1) Preparation of lipid library

As described in NPL 10 and PTLs 1 and 2, a library consisting of 14 types of pH-sensitive cationic lipids was synthesized and whose characteristics were evaluated. The names of the screened lipids were CL1A6, CL1C6, CL1D6, CL1H6, CL4C6, CL4D6, CL4F6, CL4G6, CL4H6, CL7A6, CL7H6, CL15A6, CL15F6, and CL15H6. "CL" means a cationic lipid followed by a number (1, 4, 7 or 15) representing the structure of the hydrophilic head (see FIG. 1), followed by an alphabet (A-H) representing the structure of the distal region of the hydrophobic tail (see FIG. 1), and the last number (6) representing the length of the proximal region of the hydrophobic tail. FIG. 1 illustrates a specific structure of the pH-sensitive cationic lipid.
[NPL 10] Yusuke Sato, et al., "Understanding structure-activity relationships of pH-sensitive cationic lipids facilitates the rational identification of promising lipid nanoparticles for delivering siRNAs in vivo", Journal of Control Release, Vol. 295, pp. 140-152.

Among the 14 pH-sensitive cationic lipids described above, only the preparation of the following is described: CL15A6 ((14z,17z)-5-hydroxy-5-((9z,12z)-octadeca-9,12-dien-1-yl)tricosa-14,17-dien-1-yl 1-methylpiperidine-4-carboxylate), CL15H6 (7-hydroxy-7-(4-((1-methylpiperidine-4-carbonyl)oxy)butyl)tridecane-1,13-diyl dioleate), CL15F6 (7-hydroxy-7-(4-((1-methylpiperidine-4-carbonyl)oxy)butyl)tridecane-1,13-diyl bis(2-hexyldecanoate)), CL1D6 (7-(4-(dimethylamino)butyl)-7-hydroxytridecane-1,13-diyl dipalmitate), CL1H6 (7-(4-(dimethylamino)butyl)-7-hydroxytridecane-1,13-diyl dioleate).

### (2) Synthesis of CL15A6

In 190 ml of tetrahydrofuran (THF) cooled to 4°C, 2.73g (72 mmol) of lithium aluminium hydride was suspended. To the mixture, 10g (36 mmol) of linoleic acid was added dropwise and stirred for 10 minutes. Thereafter, the mixture was refluxed overnight while being heated in an oil bath. After cooling, 100 mL of 1 mol/L aqueous sodium hydroxide solution was added to stop the reaction. Then, 100 ml of ethyl acetate was added to dilute, the resulting solution was filtered, and the filtrate was washed with a saturated aqueous solution of sodium bicarbonate. Subsequently, the organic layer was collected and dehydrated by adding anhydrous sodium sulfate thereto. After filtering, the solvent was distilled off using a rotary evaporator to obtain a crude product. The crude product was purified by silica gel chromatography (eluting solvent composed of hexane and ethyl acetate (continuous gradient)) to obtain 8.68 g (32.6 mmol) of (9z,12z)-octadecadien-1-ol as a colorless oil.

After 8.68g (32.6 mmol) of (9z,12z)-Octadecadien-1-ol was dissolved in 100 ml of dichloromethane, 366 mg (3.26 mmol) of N,N-dimethyl-4-aminopyridine (DMAP), 6.8 mL (48.9 mmol) of triethylamine (TEA) were added. Subsequently, 3.03 mL (39.1 mmol) of methanesulfonyl chloride diluted with 50 mL of dichloromethane was added dropwise using a dropping funnel, and the mixture was stirred at room temperature overnight. The reaction liquid was collected and washed with a saturated aqueous solution of sodium bicarbonate. Subsequently, anhydrous sodium sulfate was added to the organic layer and dried. After filtering, the solvent was distilled off using a rotary evaporator to obtain a crude product. The crude product was purified by silica gel chromatography (eluting solvent composed of hexane and ethyl acetate (continuous gradient)) to obtain 10.64 g (30.9 mmol) of (9z,12z)-octadecadiene-1-methanesulfonate as a colorless oil.

After dissolving 10.64 g of (9z,12z)-octadecadiene-1-methanesulfonate in 140 ml of diethyl ether, 16.0 g (61.8 mmol) of magnesium bromide ethyl etherate was added and the resulting mixture was stirred at room temperature overnight. The reaction liquid was collected and washed with 100 mL of a saturated aqueous solution of sodium bicarbonate. Subsequently, anhydrous sodium sulfate was added to the organic layer and dried. After filtration, the solvent was distilled off using a rotary evaporator to obtain a crude product. The crude product was purified by silica gel chromatography (eluting solvent composed of hexane and ethyl acetate (continuous gradient)) to obtain 8.85 g (26.9 mmol) of 18-bromo-octadeca-(6z,9z)-diene were obtained as a colorless oil.

In 1.5 mL of diethyl ether, 0.50 g (1.52 mmol) of 18-bromo-octadeca-(6z,9z)-diene was dissolved and 609 mg (25.1 mmol) of magnesium shavings were added, followed by iodine 1 flake. The resulting mixture was allowed to stand at room temperature for 10 minutes, stirred while being heated to 45°C in an oil bath, and 5.0 g (15.2 mmol) of 18-bromo-octadeca-(6z,9z)-diene dissolved in 6 mL of diethyl ether was added dropwise. The reaction was performed at 45°C for 1 hour and then cooled to room temperature. Subsequently, 300µL (3.23 mmol) of δ-valerolactone was added and the mixture was allowed to react at room temperature for 1 hour. Then, the mixture was cooled to 4°C and filtered, and the filtrate was washed with a saturated aqueous solution of sodium bicarbonate. Subsequently, anhydrous sodium sulfate was added to the organic layer and dried. After filtration, the solvent was distilled off using a rotary evaporator to obtain a crude product. The crude product was purified by silica gel chromatography (eluting solvent composed of hexane and ethyl acetate (continuous gradient)) to obtain 1.64 g (2.73 mmol) of 4-[(9z,12z)-octadecadienyl]-(13z,16z)-tricosadiene-1,4-diol as a colorless oil.

In 10 mL of 1,2-dichloroethane, 842 mg (1.40 mmol) of 4-[(9z,12z)-octadecadienyl]-(13z,16z)-tricosadiene-1,4-diol was dissolved. To this solution were added 200 mg (1.40 mmol) of 1-methyl-4-carboxylic acid, 383 mg (2.00 mmol) of 1-ethyl-3-(3-dimethylaminopropyl)carbodiimide hydrochloride, and 17.1 mg (0.14 mmol) of N,N-dimethyl-4-aminopyridine, and the resulting mixture was stirred at room temperature overnight. The mixture was then diluted with 50 ml of ethyl acetate and then separated and washed with a 1N aqueous sodium hydroxide solution and saturated saline. Subsequently, anhydrous sodium sulfate was added to the organic layer after washing, and the organic layer was dehydrated. After filtration, the solvent was distilled off using a rotary evaporator to obtain a crude product. The crude product was purified by silica gel chromatography (eluting solvent composed of dichloromethane and methanol (continuous gradient)) to obtain 877 mg (1.21 mmol) of (14z,17z)-5-hydroxy-5-((9z,12z)-octadeca-9,12-dien-1-yl)tricosa-14,17-dien-1-yl 1-methylpiperidine-4-carboxylate (CL15A6) as a colorless oil.

### (3) Synthesis of CL15H6

In 150 ml of 1,2-dichloroethane, 20.0 g (110.5 mmol) of 6-bromohexan-1-ol was dissolved and cooled to 4°C. After 18.0 g (120 mmol) of tert-butyldimethylchlorosilane (TBSCl) was added, 19.5 mL (140 mmol) of triethylamine (TEA) was added dropwise, and the resulting mixture was stirred at room temperature overnight. The solvent was distilled off using a rotary evaporator and suspended by adding 300 ml of hexane, and the insoluble matter was removed by celite filtration to obtain a crude product. The crude product was purified by silica gel chromatography (eluting solvent composed of hexane and ethyl acetate (continuous gradient)) to obtain 26.0 g (88.0 mmol) of ((6-bromohexyl)oxy)(tert-butyl)dimethylsilane as a colorless oil.

To 4 ml of diethyl ether, 1.2 g (4.06 mmol) of ((6-bromohexyl)oxy)(tert-butyl)dimethylsilane was dissolved and 2.43 g (100 mmol) of magnesium shavings was added, followed by iodine 1 flake. The resulting mixture was allowed to stand at room temperature for 10 minutes, stirred while being heated to 40°C in an oil bath, and 24.8 g (83.94 mmol) of ((6-bromohexyl)oxy)(tert-butyl)dimethylsilane dissolved in 21 ml of diethyl ether was added dropwise. After reacting at 40°C for 2 hours, the resultant was cooled to 4°C. Subsequently, 3.67 mL (39.6 mmol) of δ-valerolactone was added and the mixture was allowed to react at room temperature overnight. Then, the mixture was cooled to 4°C and 5% sulfuric acid was added dropwise to dissolve the residual magnesium. The reaction liquid was diluted with diethyl ether and then separated and washed with water and saturated saline. Subsequently, anhydrous sodium sulfate was added to the organic layer after washing, and the organic layer was dehydrated. After filtration, the solvent was distilled off using a rotary evaporator to obtain a crude product. The crude product was purified by silica gel chromatography (eluting solvent composed of hexane and ethyl acetate (continuous gradient)) to obtain 14.0 g (26.3 mmol) of 11-((tert-butyldimethylsilyl)oxy)-5-(6-((tert-butyldimethylsilyl)oxy)hexyl)undecane-1,5-diol as a colorless oil.

In 50 mL of dichloromethane, 5.33 g (10.0 mmol) of 11-((tertbutyldimethylsilyl)oxy)-5-(6-((tert-butyldimethylsilyl)oxy)hexyl)undecane-1,5-diol was dissolved, and 122 mg (1.0 mmol) of N,N-dimethyl-4-aminopyridine (DMAP) and 2.16 g (12.0 mmol) of 1-methylpiperidine-4-carboxylic acid hydrochloride were added. Subsequently, 2.49 g (13.0 mmol) of 1-(3-dimethylaminopropyl)-3-ethylcarbodiimide hydrochloride (EDCI) was slowly added and the mixture was allowed to react at room temperature overnight. The solvent was distilled off using a rotary evaporator, the resultant was suspended in ethyl acetate, and the insoluble matter was removed by filtration. The filtrate was separated and washed with a 0.5N aqueous sodium hydroxide solution and saturated saline. Subsequently, anhydrous sodium sulfate was added to the organic layer after washing, and the organic layer was dehydrated. After filtration, the solvent was distilled off using a rotary evaporator to obtain a crude product. The crude product was purified by silica gel chromatography (eluting solvent composed of dichloromethane and methanol (continuous gradient)) to obtain 5.01 g (7.61 mmol) of 11-((tertbutyldimethylsilyl)oxy)-5-(6-((tert-butyldimethylsilyl)oxy)hexyl)-5-hydroxyundecyl 1-methylpiperidine-4-carboxylate as a colorless oil.

To 5.01 g (7.61 mmol) of 11-((tert-butyldimethylsilyl)oxy)-5-(6-((tert-(butyldimethylsilyl)oxy)hexyl)-5-hydroxyundecyl 1-methylpiperidine-4-carboxylate, 1.43 mL (25 mmol) of acetic acid and 20 mL of a 1.0 M solution of tetrabutylammonium fluoride in tetrahydrofuran were added and the mixture was allowed to react at room temperature overnight. The solvent was distilled off using a rotary evaporator and purified by reverse phase silica gel chromatography (eluting solvent composed of water (0.1% trifluoroacetic acid) and acetonitrile 0.1% trifluoroacetic acid (continuous gradient)) to obtain 2.34 g (5.45 mmol) of 5,11-dihydroxy 5-(6-hydroxyhexyl)undecyl 1-methylpiperidine-4-carboxylate as a pale yellow oil.

In 10 mL of dichloromethane, 430 mg (1.00 mmol) of 5,11-dihydroxy-5-(6-hydroxyhexyl)undecyl 1-methylpiperidine-4-carboxylate was dissolved. Subsequently, 706 mg (2.50 mmol) of oleic acid, 24.4 mg (0.20 mmol) of DMAP, and 671 mg (3.5 mmol) of EDCI were added and the mixture was allowed to react at room temperature for 2 hours. The solvent was distilled off using a rotary evaporator, the resultant was suspended in ethyl acetate, and the insoluble matter was removed by filtration. The filtrate was separated and washed with a 0.5N aqueous sodium hydroxide solution and saturated saline. Subsequently, anhydrous sodium sulfate was added to the organic layer after washing, and the organic layer was dehydrated. After filtration, the solvent was distilled off using a rotary evaporator to obtain a crude product. The crude product was purified by silica gel chromatography (eluting solvent composed of dichloromethane and methanol (continuous gradient)) to obtain 569 mg (0.594 mmol) of 7-hydroxy-7-(4-((1-methylpiperidine-4-carbonyl)oxy)butyl)tridecane-1,13-diyl dioleate (CL15H6) as a pale yellow oil.

### (4) Synthesis of CL15F6

To 85.9 mg (0.20 mmol) of 5,11-dihydroxy-5-(6-hydroxyhexyl)undecyl 1-methylpiperidine-4-carboxylate in 1.5 mL of dichloromethane, 220 mg (0.80 mmol) of 2-hexyldecanoyl chloride was added and cooled on ice. To the mixture, 139 µL (1.00 mmol) of triethylamine (TEA) was added, and the reaction mixture was stirred at room temperature overnight. The solvent was distilled off using a rotary evaporator, the resultant was suspended in ethyl acetate, and the insoluble matter was removed by filtration. The filtrate was separated and washed with a 0.5N aqueous sodium hydroxide solution and saturated saline. Subsequently, anhydrous sodium sulfate was added to the organic layer after washing, and the organic layer was dehydrated. After filtration, the solvent was distilled off using a rotary evaporator to obtain a crude product. The crude product was purified by silica gel chromatography (eluting solvent composed of dichloromethane and methanol (continuous gradient)) to obtain 116 mg of (7-hydroxy-7-(4-((1-methylpiperidine-4-carbonyl)oxy)butyl)tridecane-1,13-diyl bis(2-hexyldecanoate)) (CL15F6) as a pale yellow oil.

### (5) Synthesis of CL1D6

In 150 ml of 1,2-dichloroethane, 20.0 g (110.5 mmol) of 6-bromohexan-1-ol was dissolved and cooled to 4°C. After adding 18.0 g (120 mmol) of tert-butyldimethylchlorosilane (TBSCl), 19.5 mL (140 mmol) of triethylamine (TEA) was added dropwise, and the resulting mixture was stirred at room temperature overnight. The solvent was distilled off using a rotary evaporator and suspended by adding 300 mL of hexane, and the insoluble matter was removed by celite filtration to obtain a crude product. The crude product was purified by silica gel chromatography (eluting solvent composed of hexane and ethyl acetate (continuous gradient)) to obtain 26.0 g (88.0 mmol) of ((6-bromohexyl)oxy)(tert-butyl)dimethylsilane as a colorless oil.

To 4 ml of diethyl ether, 1.2 g (4.06 mmol) of ((6-bromohexyl)oxy)(tert-butyl)dimethylsilane was dissolved and 2.43 g (100 mmol) of magnesium shavings were added, followed by iodine 1 flake. The resulting mixture was allowed to stand at room temperature for 10 minutes, stirred while being heated to 40°C in an oil bath, and 24.8 g (83.94 mmol) of ((6-bromohexyl)oxy)(tert-butyl)dimethylsilane dissolved in 21 mL of diethyl ether was added dropwise. After reacting at 40°C for 2 hours, the resultant was cooled to 4°C. Subsequently, 3.67 mL (39.6 mmol) of δ-valerolactone was added and the mixture was allowed to react at room temperature overnight. Then, the mixture was cooled to 4°C and 5% sulfuric acid was added dropwise to dissolve the residual magnesium. The resultant was diluted with diethyl ether and the organic layer was separated and washed with water and saturated saline. Subsequently, anhydrous sodium sulfate was added to the organic layer, and the organic layer was dehydrated. After filtration, the solvent was distilled off using a rotary evaporator to obtain a crude product. The crude product was purified by silica gel chromatography (eluting solvent composed of hexane and ethyl acetate (continuous gradient)) to obtain 14.0 g (26.3 mmol) of 11-((tert-butyldimethylsilyl)oxy)-5-(6-((tert-butyldimethylsilyl)oxy)hexyl)undecane-1,5-diol as a colorless oil.

In 50 mL of dichloromethane, 14.0 g (26.3 mmol) of 11-((tertbutyldimethylsilyl)oxy)-5-(6-((tert-butyldimethylsilyl)oxy)hexyl)undecane-1,5-diol was dissolved, and 321 mg (2.63 mmol) of N,N-dimethyl-4-aminopyridine (DMAP) and 5.50 mL (39.5 mmol) of diisopropylethylamine (DIPEA) were added thereto, followed by cooling to 4°C. Subsequently, 6.02 g (31.6 mmol) of p-toluenesulfonyl chloride (pTsCl) was slowly added and the mixture was allowed to react at room temperature overnight. The solvent was distilled off using a rotary evaporator, and the resultant was suspended in ethyl acetate and separated and washed with water and saturated saline. Subsequently, anhydrous sodium sulfate was added to the organic layer and dried. After filtration, the solvent was distilled off using a rotary evaporator to obtain a crude product. The crude product was purified by silica gel chromatography (eluting solvent composed of hexane and ethyl acetate (continuous gradient)) to obtain 12.4 g (28.0 mmol) of 11-((tert-butyldimethylsilyl)oxy)-5-(6-((tert-butyldimethylsilyl)oxy)hexyl)-5-hydroxyundecyl 4-methylbenzenesulfonate as a colorless oil.

To 50 mL of a 2.0 M solution of dimethylamine in tetrahydrofuran (THF), 8.78 g (12.78 mmol) of 11-((tert-butyldimethylsilyl)oxy)-5-(6-((tertbutyldimethylsilyl)oxy)hexyl)-5-hydroxyundecyl 4-methylbenzenesulfonate was added, and the mixture was allowed to react at room temperature for 6 days. The solvent was distilled off using a rotary evaporator, and the resultant was suspended in ethyl acetate and separated and washed with a saturated aqueous solution of sodium bicarbonate and saturated saline. Subsequently, anhydrous sodium sulfate was added to the organic layer and dried. After filtration, the solvent was distilled off using a rotary evaporator to obtain a crude product. The crude product was purified by silica gel chromatography (eluting solvent composed of dichloromethane and methanol (continuous gradient)) to obtain 6.20 g (11.07 mmol) of 11-(4-(dimethylamino)butyl)-2,2,3,3,19,19,20,20-octamethyl-4,18-dioxa-3,19-disilahenicosan-11-ol as a colorless oil.

To 6.20 g (11.07 mmol) of 11-(4-(dimethylamino)butyl)-2,2,3,3,19,19,20,20-octamethyl-4,18-dioxa-3,19-disilahenicosan-11-ol, 1.90 mL (33.21 mmol) of acetic acid and 24.4 mL of a 1.0 M solution of tetrabutylammonium fluoride in tetrahydrofuran were added, and the mixture was allowed to react at room temperature for 2 hours. The solvent was distilled off using a rotary evaporator and purified by reverse phase silica gel chromatography (eluting solvent composed of water (0.1% trifluoroacetic acid) and acetonitrile 0.1% trifluoroacetic acid (continuous gradient)) to obtain 2.86 g (8.63 mmol) of 7-(4-(dimethylamino)butyl)tridecane-1,7,13-triol as a pale yellow oil.

In 5 mL of dichloromethane, 431 mg (1.30 mmol) of 7-(4-(dimethylamino)butyl)tridecane-1,7,13-triol was dissolved, and 800 mg (3.12 mmol) of palmitic acid and 31.8 mg (0.26 mmol) of N,N-dimethyl-4-aminopyridine (DMAP) were added thereto, followed by 748 mg (3.90 mmol) of 1-(3-dimethylaminopropyl)-3-ethylcarbodiimide hydrochloride (EDCI), and the mixture was allowed to react at room temperature overnight. The solvent was distilled off using a rotary evaporator, the resultant was suspended in ethyl acetate, and the insoluble matter was removed by filtration. The filtrate was separated and washed with a 0.5N aqueous sodium hydroxide solution and saturated saline. Subsequently, anhydrous sodium sulfate was added to the organic layer and dried. After filtration, the solvent was distilled off using a rotary evaporator to obtain a crude product. The crude product was purified by silica gel chromatography (eluting solvent composed of dichloromethane and methanol (continuous gradient)) to obtain 557 mg (0.689 mmol) of 7-(4-(dimethylamino)butyl)-7-hydroxytridecane-1,13-diyl dipalmitate (CL1D6) as a pale yellow oil.

### (6) Synthesis of CL1H6

In 20 mL of dichloromethane, 1.99 g (6.0 mmol) of 7-(4-(dimethylamino)butyl)tridecane-1,7,13-triol was dissolved, and 4.07 g (14.4 mmol) of oleic acid and 147 mg (1.20 mmol) of N,N-dimethyl-4-aminopyridine (DMAP) were added, followed by 3.45 g (18.0 mmol) of 1-(3-dimethylaminopropyl)-3-ethylcarbodiimide hydrochloride (EDCI), and the mixture was allowed to react at room temperature overnight. The solvent was distilled off using a rotary evaporator, the resultant was suspended in ethyl acetate, and the insoluble matter was removed by filtration. The filtrate was separated and washed with a 0.5N aqueous sodium hydroxide solution and saturated saline. Subsequently, anhydrous sodium sulfate was added to the organic layer and dried. After filtration, the solvent was distilled off using a rotary evaporator to obtain a crude product. The crude product was purified by silica gel chromatography (eluting solvent composed of dichloromethane and methanol (continuous gradient)) to obtain 2.56 g (2.98 mmol) of 7-(4-(dimethylamino)butyl)-7-hydroxytridecane-1,13-diyl dioleate (CL1H6) as a pale yellow oil.

### (7) Preparation and characteristics evaluation of lipid nanoparticle

Lipid nanoparticles encapsulating mRNA were prepared using a microchannel device (iLiNP device) described in NPL 11. Using the iLiNP device, an ethanol solution (organic phase) of a lipid composition containing the above-mentioned pH-sensitive cationic lipid as the main component was rapidly mixed with an acidic buffer solution (pH 4) (aqueous phase) containing mRNA (total flow rate: 500 µL/min) to prepare lipid nanoparticles. In addition to the pH-sensitive cationic lipid, the lipid composition also contained a phospholipid (helper phospholipid), cholesterol, and a PEG-modified lipid. The physicochemical properties of the lipid nanoparticles were controlled by adjusting the type of lipid, the nitrogen/phosphate (N/P) ratio, the type of aqueous buffer, and the flow rate ratio of the aqueous phase to the organic phase. The lipid nanoparticles were then concentrated with an Amicon filter unit, and the medium was exchanged into 10 mM HEPES buffer (pH = 7.4) or PBS (pH = 7.4) to place the lipid nanoparticles under physiological conditions.
[NPL 11] Niko Kimura, et al., "Development of the iLiNP Device: Fine Tuning the Lipid Nanoparticle Size within 10 nm for Drug Delivery", ACS Omega, Vol. 3, pp. 5044-5051

The average particle size, particle size distribution, and zeta potential of the lipid nanoparticles were measured using a Zetasizer Nano ZS (Malvern Instruments Ltd, UK) by dynamic light scattering and the form of lipid nanoparticle was measured using a transmission electron-microscope as described in NPL 12. The efficiency of mRNA encapsulation was estimated by RiboGreen fluorometry as described in NPL 13. Although the respective values vary depending on the type of pH-sensitive cationic lipid, the composition of the lipids constituting the lipid nanoparticles, the preparation conditions of the lipid nanoparticles, and the like, the average particle size of the lipid nanoparticles was in the range of 80 to 350 nm, the PDI was in the range of 0.05 to 0.4, the zeta potential was in the range of -16 to +5 mV, and the efficiency of mRNA encapsulation was 75 to 95%.
[NPL 12] Mahmoud A. Younis, et al., "A Multifunctional Lipid-Based Nanodevice for the Highly Specific Codelivery of Sorafenib and Midkine siRNA to Hepatic Cancer Cells", Molecular Pharmaceutics, Vol. 16, pp. 4031-4044
[NPL 13] Mahmoud M. Abd Elwakil, et al., "Engineered ε-decalactone lipomers bypass the liver to selectively in vivo deliver mRNA to the lungs without targeting ligands", Materials Horizons, Vol. 8, pp. 2251-2259

The apparent pKa of the lipid nanoparticles was also measured using 6-p-toluidino-2-naphthalenesulfonic acid (TNS) (see NPL 14). Specifically, in each well of a 96-well plate, lipid (0.5 mM) of the lipid nanoparticles and TNS (0.6 mM) were added to 200 µL of a buffer solution and mixed. The buffer solution was 20 mM citrate buffer (pH3.50 to 5.50), 20 mM sodium phosphate buffer (pH6.00 to 8.00), or 20 mM Tris-HCl buffer (pH8.00 to 9.00), and each buffer solution contained 150 mM NaCl. At this time, the final concentration of lipid was adjusted to 30 µM and the final concentration of TNS was adjusted to 6 µM. Fluorescence (excitation wavelength 321nm, fluorescence wavelength 447nm) was then measured at 37°C using a plate reader (Enspire 2300 multilabel reader, Perkin Elmer). The pKa was defined as the pH value showing 50% of the maximum fluorescence intensity.
[NPL 14] Nour Shobaki, Yusuke Sato, and Hideyoshi Harashima, "Mixing lipids to manipulate the ionization status of lipid nanoparticles for specific tissue targeting", International Journal of Nanomedicine, Vol. 13, pp. 8395-8410

The relationship between the type of pH-sensitive cationic lipid used and the apparent pKa of lipid nanoparticles is shown below in Table 1.

**[Table 1]**

| pH-sensitive cationic lipid | Apparent pKa |
|---|---|
| CL1A6 | 8.20 |
| CL1C6 | 8.30 |
| CL1D6 | 8.15 |
| CL1H6 | 8.00 |
| CL4C6 | 6.30 |
| CL4D6 | 6.40 |
| CL4F6 | 6.00 |
| CL4G6 | 6.28 |
| CL4H6 | 6.25 |
| CL7A6 | 5.85 |
| CL7H6 | 5.96 |
| CL15A6 | 7.30 |
| CL15F6 | 6.75 |
| CL15H6 | 7.24 |

### 2. Evaluation in vitro

Fourteen types of lipid nanoparticles prepared were screened with activated hepatic stellate cell line LX-2 (Merck (Germany) and hepatocyte line FL83B (ATCC, USA).

Twenty-four hours prior to the experiment, cells were seeded in 6-well plates at 1×10⁵ cells/well and incubated at 37°C. The medium was aspirated and the cells were washed with (-) PBS and then treated with lipid nanoparticles encapsulating EGFP mRNA (TriLink BioTechnologies, USA) in 1.5 ml of growth medium for 24 hours. The amount of mRNA per well was 250 ng. Cells were then washed with (-) PBS and incubated in fresh medium for an additional 21 hours. The median fluorescence intensity of EGFP and the percentage of transfected cells were evaluated by fluorescence activated cell sorting (FACS) using a flow cytometer (Cytoflex, Beckman Coulter, USA).

In addition, the cytotoxicity of the prepared lipid nanoparticles against cells was evaluated by MTT assay (Abcam, UK) as described in NPL 12.

FIGS. 2A to 2C are graphs (N=3) showing the results of screening using LX-2 cells for EGFP mRNA encapsulating lipid nanoparticles prepared by using various pH-sensitive cationic lipids. FIG. 2A is a graph showing the relationship between the type of pH-sensitive cationic lipid and mRNA delivery efficiency (mean value ± standard deviation), FIG. 2B is a graph showing the relationship between the type of pH-sensitive cationic lipid and transfection efficiency (mean value ± standard deviation) (***: P<0.001), and FIG. 2C is a graph showing the relationship between the type of pH-sensitive cationic lipid and the cell viability (mean value ± standard deviation).

As shown in FIG. 2A, the efficiency of mRNA delivery to LX-2 cells significantly differed depending on the type of pH-sensitive cationic lipid. On the other hand, as shown in FIG. 2B, the percentage of transfected cells was almost the same depending on the type of pH-sensitive cationic lipid. As shown in FIG. 2C, most pH-sensitive cationic lipids were tolerated by LX-2 cells, and the cell viability was 80% or more.

Five types of pH-sensitive cationic lipids (CL1D6, CL1H6, CL15A6, CL15F6, and CL15H6) with high mRNA delivery efficiency were evaluated for selectivity against LX-2 cells (hepatic stellate cells) and FL83B cells (hepatocytes).

FIGS. 3A to 3C are graphs (N=3) showing the selectivity of mRNA delivery to LX-2 and FL83B cells for EGFP mRNA encapsulating lipid nanoparticles prepared by using various pH-sensitive cationic lipid. FIG. 3A is a graph showing the relationship between the type of pH-sensitive cationic lipid and the mRNA delivery efficiency (mean value ± standard deviation) (***: P<0.001, #: P<0.0001), FIG. 3B is a graph showing the relationship between the type of pH-sensitive cationic lipid and the transfection efficiency (***: P<0.001, #: P<0.0001), FIG. 3C is a graph showing the relationship between the pKa of pH-sensitive cationic lipid and the mRNA delivery efficiency (mean value ± standard deviation).

As shown in FIGS. 3A and 3B, all five selected lipid showed higher selectivity for LX-2 cells compared to FL83B cells. As shown in FIG. 3C, the pKa of lipids with high mRNA delivery efficiency to FL83B cells was 5.8 to 6.7, while the pKa of lipids with high mRNA delivery efficiency to LX-2 cells was 6.7 or more and less than 8.2 (6.75 to 8.15). This difference may be related not only to the endosomal escape capability of the lipid nanoparticles but also to changes in the protein corona formed in serum.

As shown in FIGS. 3A to 3C, CL15A6 showed promising mRNA delivery efficiency and transfection efficiency for HSC in vitro. Therefore, CL15A6 was selected as the model lipid of the optimization process in vivo.

### 3. Evaluation in vivo

Thioacetamide (TAA (TCI, Japan))-induced liver fibrosis models were generated in 4-week-old male BALB/c mise (Sankyo laboratories,, Japan). Specifically, TAA dissolved in saline was injected intraperitoneally at an amount of 200 mg/kg of TAA per kg of body weight. This was repeated every 3 days for a total of 10 times. The livers of the liver fibrosis model mice were examined by histopathology and hydroxyproline quantitative assay (Cell Biolabs Inc, USA), and the activation of hepatic stellate cells (HSCs) was confirmed by quantitative real-time polymerase chain reaction (qRT-PCR).

The lipid nanoparticles prepared by using CL15A6 encapsulated the above-described EGFP mRNA or firefly luciferase (FLuc) mRNA (TriLink BioTechnologies, USA), and the lipid nanoparticles were administered intravenously to mice via the tail vein so that the amount of RNA per kg of body weight was 1 mg/kg. Twenty-four hours after the administration, hepatic stellate cells and hepatocytes were collected from the livers of the mice as described in NPL 15. For EGFP mRNA, FACS was used to evaluate delivery efficiency and transfection efficiency, and for FLuc mRNA, the luciferase assay was used to evaluate delivery efficiency as described in NPL 16.
[NPL 15] Ingmar Mederacke, et al., "High-yield and high-purity isolation of hepatic stellate cells from normal and fibrotic mouse livers", Nature Protocols, Vol. 10, pp. 305-315
[NPL 16] Ikramy A. Khalil, et al., "Synergism between a cell penetrating peptide and a pH-sensitive cationic lipid in efficient gene delivery based on double-coated nanoparticles", Journal of Controlled Release, Vol. 275, pp. 107-116

First, the microchannel device (iLiNP device) was operated under different conditions to prepare lipid nanoparticles with different average particle sizes. Specifically, lipid nanoparticles with an average particle size of more than 150 nm (e.g., 180 nm), lipid nanoparticles with an average particle size of 100 to 150 nm (e.g., 130 nm), and lipid nanoparticles with an average particle size of less than 100 nm (e.g., 90 nm) were prepared. The prepared lipid nanoparticles were administered intravenously to mice with liver fibrosis, and the mRNA delivery efficiency and transfection efficiency with respect to hepatic stellate cells and hepatocytes were evaluated 24 hours later.

FIG. 4A is a graph showing the relationship between the particle size of lipid nanoparticles prepared by using CL15A6 and the mRNA delivery efficiency (mean value ± standard deviation) (N=3, #: P<0.0001), and FIG. 4B is a graph showing the relationship between the particle size of lipid nanoparticle prepared by using CL15A6 and the transfection efficiency (mean value ± standard deviation) (N=3, #: P<0.0001). As shown in these graphs, when the particle size of lipid nanoparticles was reduced, both the delivery efficiency and the transfection efficiency of mRNA to hepatic stellate cells were linearly improved. This suggests that lipid nanoparticles prepared by using CL15A6 penetrate the microenvironment rich in fibrotic liver stroma.

Next, lipid nanoparticles with different nitrogen/phosphate (N/P) ratios were prepared by mixing a lipid composition containing CL15A6 with mRNA at different ratios. Specifically, lipid nanoparticles with N/P ratios of 4, 8, 12, 16, and 20 were prepared. The prepared lipid nanoparticles were intravenously administered to mice with liver fibrosis, and the mRNA delivery efficiency and transfection efficiency with respect to hepatic stellate cells and hepatocytes were evaluated 24 hours later.

FIG. 5A is a graph showing the relationship between the N/P ratio of the lipid nanoparticles prepared by using CL15A6 and the mRNA delivery efficiency (mean value ± standard deviation) (N=3, ***: P<0.001, #: P<0.0001), and FIG. 5B is a graph showing the relationship between the N/P ratio of the lipid nanoparticles prepared by using CL15A6 and the transfection efficiency (mean value ± standard deviation) (N=3, #: P<0.0001). As shown in these graphs, when the N/P ratio was increased from 4 to 8, the delivery of mRNA to hepatic stellate cells was significantly improved. This is considered to be due to the improved endosomal escape capability of the lipid nanoparticles. It is also considered that lipid nanoparticles were more likely to penetrate the interstitial barrier of the fibrous liver because average particle size of the lipid nanoparticles was decreased to 80 nm. The preferred range of the N/P ratio for hepatic stellate cells was 6 to 14 (optimal range of 8 to 12) and the optimal value of the N/P ratio for hepatic cells was 12. On the other hand, the delivery efficiency decreased when the N/P ratio was increased from 16 to 20. The decrease in delivery efficiency may indicate a decrease in the release of mRNA from the lipid nanoparticles due to the high cation content.

Next, lipid nanoparticles containing different types of helper phospholipids were prepared by changing the type of helper phospholipid in the lipid composition containing CL15A6. As the unsaturated phosphatidylethanolamine, 1,2-dioleoyl-sn-glycero-3-phosphoethanolamine (DOPE) was used. As the unsaturated phosphatidylcholine, 1,2-dioleoyl-sn-glycero-3-phosphocholine (DOPC) was used. As the saturated phospholipid, 1,2-distearoyl-sn-glycero-3-phosphocholine (DSPC) was used. The prepared lipid nanoparticles were intravenously administered to mice with liver fibrosis, and the mRNA delivery efficiency and transfection efficiency with respect to hepatic stellate cells and hepatocytes were evaluated 24 hours later.

FIG. 6A is a graph showing the relationship between the type of helper phospholipid of the lipid nanoparticles prepared by using CL15A6 and the mRNA delivery efficiency (mean value ± standard deviation) (N=3, ***: P<0.001, #: P<0.0001), and FIG. 6B is a graph showing the relationship between the type of helper phospholipid of the lipid nanoparticles prepared by using CL15A6 and the transfection efficiency (mean value ± standard deviation) (N=3, *: P<0.05, #: P<0.0001). As shown in these graphs, DOPE significantly improved mRNA delivery to hepatic stellate cells compared to other helper phospholipids. This is considered to be due to the improved endosomal escape capability of the lipid nanoparticles.

Next, lipid nanoparticles containing helper phospholipid (DOPE) at different molar percentages were prepared by changing the molar ratio of the helper phospholipid (DOPE). The prepared lipid nanoparticles were intravenously administered to mice with liver fibrosis, and the mRNA delivery efficiency and transfection efficiency with respect to hepatic stellate cells and hepatocytes were evaluated 24 hours later.

FIG. 7A is a graph showing the relationship between the percentage of DOPE in the lipid nanoparticles prepared by using CL15A6 and the mRNA delivery efficiency (mean value ± standard deviation) (N=3, ***: P<0.001), and FIG. 7B is a graph (N=3) showing the relationship between the percentage of DOPE in the lipid nanoparticles prepared by using CL15A6 and the transfection efficiency (mean value ± standard deviation). As shown in these graphs, when the percentage of DOPE was 20 to 30 mol%, especially when it was 30 mol%, the delivery of mRNA to hepatic stellate cells was significantly improved. On the other hand, when the percentage of cholesterol was reduced to set the percentage of DOPE to 40 mol%, the delivery of mRNA to hepatic stellate cells was reduced. This suggests that mRNA delivery efficiency changes due to differences in the endosomal escape capability and/or stabilities of the lipid nanoparticles.

Next, lipid nanoparticles containing PEG-modified lipid (DMG-PEG2K) at different molar percentages were prepared by changing the molar ratio of the PEG-modified lipid, namely 1,2-dimyristoyl-rac-glycerol-3-methoxypolyethylene glycol-2000 (DMG-PEG2K), in the lipid composition containing CL15A6. The prepared lipid nanoparticles were intravenously administered to mice with liver fibrosis, and the mRNA delivery efficiency and transfection efficiency with respect to hepatic stellate cells and hepatocytes were evaluated 24 hours later.

FIG. 8A is a graph showing the relationship between the percentage of DMG-PEG2K in the lipid nanoparticles prepared by using CL15A6 and the mRNA delivery efficiency (mean value ± standard deviation) (N=3, *: P<0.05), and FIG. 8B is a graph showing the relationship between the percentage of DMG-PEG2K in the lipid nanoparticles prepared by using CL15A6 and the transfection efficiency (mean value ± standard deviation) (N=3, *: P<0.05). As shown in these graphs, when the percentage of DMG-PEG2K was 3 to 5 mol%, the efficiency of mRNA delivery to hepatic stellate cells was high. When the percentage of DMG-PEG2K was 1.5 mol%, the particle size became large (to 100 nm) and the mRNA delivery efficiency decreased. As shown in FIG. 8B, when the percentage of DMG-PEG2K was 3 to 5 mol%, 80% or more of hepatic stellate cells in vivo could be transfected. This is a very encouraging result in view of the difficult microenvironment of the liver and the difficulty of transfecting hepatic stellate cells.

From the above results, it was found that the lipids constituting the lipid membrane of the lipid nanoparticles preferably contain CL15A6, DOPE, cholesterol and PEG-modified lipid. Therefore, lipid nanoparticles containing CL15A6, DOPE, cholesterol and DMG-PEG2K in a molar ratio of 5:3:2:0.3 and having an average particle size of 80 nm (PDI<0.1) were used in the subsequent experiments. FIG. 9A is a schematic diagram showing the configuration of this lipid nanoparticle, FIG. 9B is a transmission electron microscope image of the lipid nanoparticle, and FIG. 9C is a graph showing the particle size distribution of the lipid nanoparticles.

### 4. Robustness and intrahepatic selectivity of mRNA delivery

To confirm the clinical applicability of the lipid nanoparticles prepared by using CL15A6, the efficiency of mRNA delivery to hepatic stellate cells in vivo and the robustness of intrahepatic selectivity were verified by using mRNAs that vary in length and base sequence and code for different proteins.

First, lipid nanoparticles were prepared by replacing the EGFP mRNA (996 bases, coding green fluorescent EGFP protein) used in the above experiment with FLuc mRNA (1929 bases, coding bioluminescent firefly luciferase) or mCherry mRNA(996 bases, coding red fluorescent mCherry protein). The prepared lipid nanoparticles were intravenously administered to mice with liver fibrosis, and the mRNA delivery efficiency and transfection efficiency with respect to hepatic stellate cells and hepatocytes were evaluated 24 hours later.

In addition, the delivery of mRNA in the liver was observed using a confocal laser scanning microscope (CLSM). Lipid nanoparticles incorporating mCherry mRNA (TriLink BioTechnologies, USA) were intravenously injected into mice so that the amount of RNA per kg of body weight was 1 mg/kg. 23.5 hours after the administration, 50 µg of FITC-labeled isolectin B4 (FITC-IB4) (Vector laboratories, USA) was intravenously administered to the mice to stain the blood vessels green. After 30 minutes, the liver was perfused with (-) PBS and excised. Liver tissue sections were prepared and stained with 10 µg/mL Hoechst 33342 (Thermo Fisher Scientific, USA) for 30 minutes, and the nuclei were stained blue. The tissue sections were then observed with a CLSM (Nikon, Japan), and mCherry-positive cells were visualized in red.

FIG. 10A is a graph showing the luciferase activity (mean value ± standard deviation) of hepatic stellate cells and hepatocytes in the liver after the administration of lipid nanoparticles encapsulating FLuc mRNA (N=3, #: P<0.0001). FIG. 10B is a fluorescent observation image (scale bar: 100 µm) of a liver tissue section after the administration of the lipid nanoparticles encapsulating mCherry mRNA. FIG. 10B is grayscaled, but originally the nuclei of each cell appear blue (large particles appear blue), cells expressing mCherry protein appear red (small particles around the nucleus appear red), and hepatic sinusoidal endothelial cells appear green (net-shaped continuous parts appear green).

As shown in FIGS. 10A and 10B, high mRNA delivery efficiency and intrahepatic selectivity with respires to hepatic stellate cells were maintained regardless of the type of mRNAused. In addition, as shown in FIG. 10B, the mCherry-positive cells were dispersed in the fluorescent observation image of the peri sinusoidal region of the liver, suggesting that hepatic stellate cells were transfected. Furthermore, the fact that mCherry-positive cells were observed in a region different from the FITC-stained vasculature suggests that hepatic sinusoidal endothelial cells are less likely to be transfected.

### 5. Biological safety

To further confirm the clinical applicability of the lipid nanoparticles prepared by using CL15A6, the biological safety when the dosage amount of mRNA is increased was evaluated in vivo.

In order to exclude the effects of liver fibrosis on hepatic function and tissues, the biosafety of the lipid nanoparticle prepared by using CL15A6 was evaluated in normal mice. Lipid nanoparticles containing EGFP mRNA were administered intravenously to mice at an amount of 2 mg/kg RNA per kg body weight. Twenty-four hours after the administration, the mice were euthanized, and serum and major organs were collected and analyzed in comparison with control mice treated with HEPES buffer. Aspartate transaminase (AST), alanine aminotransferase (ALT), and creatinine (CRE), which are functional markers of the liver or kidney, in plasma were colorimetrically quantified using a commercially available kit (Sigma-Aldrich), and the results were compared. In addition, the collected organs were fixed, stained with hematoxylin and eosin, and histopathologically evaluated as described in NPL 17.
[NPL 17] Mahmoud A. Younis, et al., "Ultra-small lipid nanoparticles encapsulating sorafenib and midkine-siRNA selectively-eradicate sorafenib-resistant hepatocellular carcinoma in vivo", J Control Release, Vol. 331, pp. 335-349

FIG. 11A illustrates photographs showing tissue images of the major organs of mice after the administration of the lipid nanoparticles, and FIG. 11B is a graph (N=3) showing the measured values (mean value ± standard deviation) of liver or kidney toxicity markers in the sera of the mice after the administration of the lipid nanoparticles.

As shown in FIG. 11A, no signs of injury were observed in the major organs (liver, lung, heart, and kidney). In addition, as shown in FIG. 11B, no significant increases were observed in AST, ALT, and CRE, suggesting that there was no toxicity despite the administration of high doses of mRNA.

### [Example 2]

In Example 2, an experiment was carried out to deliver siRNA to hepatic stellate cells.

### 1. Preparation and characteristics evaluation of lipid nanoparticle encapsulating siRNA

### (1) Preparation of lipid nanoparticles

As a target of siRNA, the mRNA of the Smoothened (SMO) gene was selected. Patched 1 (PTCH1) protein inhibits the activation of hepatic stellate cells by suppressing SMO protein, and makes them quiescent (qHSC). When the liver is damaged, hedgehog ligands (SHH, IHH, and DHH) released from injured hepatocytes activate SMO by inhibiting PTCH1. SMO stimulates glioma-associated transcription factor (GLI) and transforms quiescent HSC (qHSC) into activated hepatic stellate cells (aHSC) that are involved in collagen production and the progression of hepatic fibrosis. The SMO gene is overexpressed in aHSC and is considered to be a characteristic marker of aHSC.

As the pH-sensitive cationic lipid constituting the lipid nanoparticles, CL15A6 and CL15H6, which showed excellent mRNA delivery efficiency and transfection efficiency with respect to hepatic stellate cells in Example 1, were selected.

Using the iLiNP device in the same manner as in Example 1, lipid nanoparticles containing the above pH-sensitive cationic lipid as a main component and encapsulating SMO-siRNA (SEQ ID NOs: 1 and 2) having the sequences shown in Table 2 below were prepared. The lipids constituting the lipid nanoparticles contained a pH-sensitive cationic lipid (CL15A6 or CL15H6), DOPE, cholesterol, and DMG-PEG2K in a molar ratio of 5:3:2:0.3.

**[Table 2]**

| SEQ ID NO | | Sequence (5'→3') |
|---|---|---|
| 1 | SMO-siRNA Sense | GGA GUC AUG ACU CUG UUC U[dT] [dT] |
| 2 | SMO-siRNA Anti-sense | AGA ACA GAG UCA UGA CUC C[dT][dT] |

| | | |
|---|---|---|
| [dT] = 2'-Deoxythymidine | | |

The average particle size, particle size distribution, and zeta potential of the lipid nanoparticles were measured in the same manner as in Example 1 to estimate the efficiency of SMO-siRNA encapsulation. The relationship between the flow rate ratio of the aqueous phase to the organic phase, the average particle size, particle size distribution, zeta potential, and SMO-siRNA encapsulation efficiency of the lipid nanoparticles prepared at the same flow rate ratio when CL15H6 was used as the pH-sensitive cationic lipid is shown in Table 3 below. In the subsequent experiments, lipid nanoparticles were prepared at a ratio of 4:1 (aqueous phase : organic phase), which provided the highest encapsulation efficiency, the smallest average particle size, and the smallest PDI.

**[Table 3]**

| Aqueous phase: organic phase | Average particle size (nm) | PDI | Zeta potential (mV) | Encapsulation efficiency (%) |
|---|---|---|---|---|
| 2:1 | 135.72 | 0.280 | -6.81 | 66.12 |
| 3:1 | 72.5 | 0.192 | -3.75 | 92.33 |
| 4:1 | 53.45 | 0.093 | -3.35 | 94.06 |
| 5:1 | 57.12 | 0.169 | -5.08 | 89.19 |
| 9:1 | 58.53 | 0.217 | -8.2 | 75.55 |

The apparent pKa of the lipid nanoparticles was measured in the same manner as in experiment 1. As a result, the pKa of the lipid nanoparticles prepared with CL15A6 as the pH-sensitive cationic lipid was 7.30, and the pKa of the lipid nanoparticles prepared with CL15H6 as the pH-sensitive cationic lipid was 7.24.

### 2. Evaluation in vivo

TAA-induced liver fibrosis model mice were prepared in the same manner as in Example 1, and SMO-siRNA-encapsulated lipid nanoparticles were intravenously administered to the mice via the tail vein so that the amount of RNA per kg of body weight was 0.5 mg/kg. 24 hours after the administration, the mouse livers were collected. The ratio of the amount of SMO mRNA to the amount of GAPDH mRNA in the liver tissue was confirmed by qRT-PCR. The SMO gene is expressed only in hepatic stellate cells in the liver.

FIG. 12 is a graph (N=3, #: P<0.0001) showing the percentage of the amount of SMO mRNA when the lipid nanoparticles prepared by using CL15A6 were administered, the percentage of the amount of SMO mRNA when the lipid nanoparticles prepared by using CL15H6 were administered, and the percentage of the amount of SMO mRNA when HEPES buffer was administered in place of lipid nanoparticles (control). As shown in this graph, the amount of SMO mRNA was significantly reduced when any of the pH-sensitive cationic lipids was used. This suggests that lipid nanoparticles prepared by using CL15A6 or CL15H6 were able to suppress the expression of the SMO gene in vivo.

The lipid nanoparticles prepared by using CL15A6 were evaluated in Example 1, and thus the lipid nanoparticles prepared by using CL15H6 were used in the subsequent experiments of Example 2.

Next, a lipid composition containing CL15H6 and SMO-siRNA were mixed in different ratios to prepare lipid nanoparticles with different nitrogen/phosphate (N/P) ratios. Specifically, lipid nanoparticles were prepared with N/P ratios of 0.5, 1, 2, 4, 8, 12, 16, and 20. The prepared lipid nanoparticles were intravenously administered to mice with liver fibrosis so that the amount of RNA per kg of body weight was 0.5 mg/kg, and the ratio of the amount of SMO mRNA to the amount of GAPDH mRNA in the liver tissue was confirmed 24 hours later.

FIG. 13 is a graph showing the relationship between the N/P ratio of lipid nanoparticles prepared by using CL15H6 and the percentage of the amount of SMO mRNA (N=3, #: P<0.0001 (N/P = 0.5 to 1, 2 against N/P = 4 to 8), ***: P<0.001 (N/P = 20 against N/P = 8), **: P<0.01 (N/P = 12 against N/P = 8)). As shown in Example 1, the preferred range of N/P ratio for mRNA delivery was 6 to 14 (optimal range 8 to 12) (see FIG. 5A), while the preferred range of N/P ratio for siRNA delivery was 4 to 8. Presumably, decreasing the N/P ratio below 4 would result in insufficient endosomal escape capability of the lipid nanoparticles, while increasing the N/P ratio above 8 would inhibit the release of siRNA in cells. In subsequent experiments, the N/P ratio was set to 4, which gave the lowest amount of SMO mRNA.

Next, the relationship between the dosage amount of SMO-siRNA and the ratio of the amount of SMO mRNA to the amount of GAPDH mRNA in the liver tissue was examined. For comparison, the relationship between the dosage amount of control siRNA (siCtrl) and the ratio of SMO mRNA to GAPDH mRNA in hepatic stellate cells was also examined.

FIG. 14 is graph showing the dose-response curve of the lipid nanoparticles prepared by using CL15H6 (N=3, #: P<0.0001). As shown in this graph, the 50% effective dose (ED₅₀) when SMO-siRNA was administered was 0.08 mg/kg or less, indicating that the lipid nanoparticles have high efficacy. In the subsequent experiments, the dosage amount of SMO-siRNA was set to 0.25 mg/kg, which was the lowest amount of SMO mRNA.

### 3. Treatment of liver fibrosis

As described above, liver fibrosis develops in response to liver damage, causing hepatocyte injury and Kupffer cell activation. Damaged hepatocytes release hedgehog ligands (Hh), which activates the SMO signal pathway in hepatic stellate cells and activates hepatic stellate cells. The activated Kupffer cells release transforming growth factor β1 (TGFβ1). The TGFβ1 activates the TGFβ1 signaling pathway in hepatic stellate cells and further activates the hepatic stellate cells. Therefore, knockdown of the SMO gene and the TGFβ1 gene at the same time is expected to inactivate HSC and make it quiescent. Therefore, it was attempted to simultaneously knockdown the SMO gene and the TGFβ1 gene using lipid nanoparticles prepared by using CL15H6.

Lipid nanoparticles encapsulating a mixture of the above SMO-siRNA and TGFβ1-siRNA at a ratio of 1:1 were prepared by using CL15H6, the above SMO-siRNA (SEQ ID NOs: 1 and 2) targeting the mRNA of the SMO gene and the TGFβ1-siRNA (SEQ ID NOs: 3 and 4) targeting the mRNA of the TGFβ1 gene and having the sequence shown in Table 4 below. The prepared lipid nanoparticles were intravenously administered to mice with liver fibrosis so that each of the amounts of SMO-siRNA and of TGFβ1-siRNA per kg of body weight was 0.25 mg/kg, and 24 hours later, the ratios of the amounts of SMO mRNA and of TGFβ1 mRNA to the amount of GAPDH mRNA in the liver tissue was confirmed.

**[Table 4]**

| SEQ ID NO | | Sequence (5'-3') |
|---|---|---|
| 3 | TGFβ1-siRNA Sense | GCU GUA CAU UGA CUU UAG G[dT] [dT] |
| 4 | TGFβ1-siRNA Anti-sense | CCU AAA GUC AAU GUA CAG C[dT] [dT] |

| | | |
|---|---|---|
| [dT] = 2'-Deoxythymidine | | |

FIG. 15 is a graph (N=3, #: P<0.0001) showing the percentage of the amount of mRNA of the target gene when the lipid nanoparticles prepared by using CL15H6 were administered, and the percentage of the amount of mRNA of the target gene when HEPES buffer was administered in place of the lipid nanoparticles (control). As shown in this graph, the SMO gene and the TGFβ1 gene could be knocked down simultaneously by combining SMO-siRNA and TGFβ1-siRNA.

Next, the lipid nanoparticles prepared by using CL15H6 and containing two types of siRNA were intravenously administered to mice with liver fibrosis five times every three days so that the amount of each type of siRNA per kg body weight was 0.25 mg/kg. The livers of the mice were collected three days after the last administration. The siRNAs used were a combination of two types of control siRNA (siCntrl), a combination of SMO-siRNA and control siRNA (SMO-siRNA), a combination of TGFβ1-siRNA and control siRNA (TGFβ1-siRNA), and a combination of SMO-siRNA and TGFβ1-siRNA (siCocktail).

FIG. 16 illustrates photographs of the livers of mice administered respective types of lipid nanoparticles. As can be seen from the photographs, the liver of the mouse administered with the combination of SMO-siRNA and TGFβ1-siRNA (siCocktail) showed an appearance closer to that of a healthy mouse than those of the mice administered with the other combinations. Specifically, in the livers of the mice administered with the other combinations, the surface of the liver had fine irregularities due to a large number of nodules present in the liver, whereas in the liver of the mouse administered with the combination of SMO-siRNA and TGFβ1-siRNA (siCocktail) had fewer nodules and a smooth surface.

Sections of the collected livers were also prepared. Some sections were stained with anti-α smooth muscle actin (α-SMA) to detect aHSC, and the percentage of aHSC in HSC was measured. Some of other sections were also stained with Masson's trichrome to detect collagen fibers and the percentage of collagen area in the field of view was measured.

FIG. 17A illustrates stained images of the liver sections of respective mice with anti-α-SMA antibodies. In FIG. 17A, CV indicates the central vein, the arrows indicate α-SMA positive cells, and the scale bar indicates 100 µm. FIG. 17B is a graph showing the percentage of aHSC in the liver of each mouse (N=5, **: P<0.01 (SMO-siRNA or TGFβ1-siRNA against siCntrl or siCocktail), #: P<0.0001 (siCocktail against siCntrl)).

FIG. 18A illustrates Masson's trichrome stained images of liver sections of respective mice. In FIG. 18A, arrows indicate collagen fibers and scale bars indicate 100 µm. FIG. 18B is a graph showing the percentage of the area of collagen in the field of view for the liver of each mouse (N=5, #: P<0.0001 (SMO-siRNA, TGFβ1-siRNA, or siCocktail against siCntrl), *P<0.05 (siCocktail against SMO-siRNA or TGFβ1-siRNA)).

As shown in FIGS. 17A and 17B, the percentage of aHSC was high in the liver of the mouse administered only control siRNA, whereas the percentage of aHSC was significantly decreased in the liver of the mouse administered SMO-siRNA or TGFβ1-siRNA. Furthermore, the percentage of aHSC was dramatically decreased in the liver of the mouse administered a combination of SMO-siRNA and TGFβ1-siRNA (siCocktail).

As shown in FIGS. 18A and 18B, collagen fibers were abundant in the liver of the mouse administered with only the control siRNA, whereas collagen fibers were dramatically reduced in the liver of a mouse administered SMO-siRNA, TGFβ1-siRNA, or a combination thereof. In particular, collagen fibrils were most reduced in the liver of the mouse administered with the combination of SMO-siRNA and TGFβ1-siRNA (siCocktail).

The concentrations of inflammatory cytokines (interleukin-6 (IL-6) and tumor-necrosis factor-alpha (TNF-alpha)) in the sera of these mice were determined by ELISA. FIG. 19A is a graph showing the concentration of IL-6 in the serum of each mouse, and FIG. 19B is a graph showing the concentration of TNF-α in the serum of each mouse (N=5, *: P<0.05, #: P<0.0001).

As shown in FIGS. 19A and 19B, the amount of inflammatory cytokines was only slightly reduced in the mouse administered with only one of SMO-siRNA or TGFβ1-siRNA, whereas the amount of inflammatory cytokines in the mouse administered with the combination of SMO-siRNA and TGFβ1-siRNA was significantly reduced to a value close to those in a healthy state. This suggests that the combination of SMO-siRNA and TGFβ1-siRNA has significant anti-inflammatory effect. Furthermore, statistical verification by two-way ANOVA suggested that SMO-siRNA and TGFβ1-siRNA have a synergistic anti-inflammatory effect against both IL-6 and TNF-α.

In addition, the concentrations of hepatic function markers (aspartate aminotransferase (AST), alanine aminotransferase (ALT), alkaline phosphatase (ALP), and total bilirubin (T-BIL)) in the sera of these mice were determined.

FIG. 20A is a graph showing the concentration of AST in the serum of each mouse (N=5, *P<0.05, ***: P<0.001 (siCocktail against siCntrl), **: P<0.01 (siCocktail against SMO-siRNA or TGFβ1-siRNA)). FIG. 20B is a graph showing the concentration of ALT in the serum of each mouse (N=5, *: P<0.05 (SMO-siRNA or TGFβ1-siRNA against siCntrl, and SMO-siRNA or TGFβ1-siRNA against siCocktail), **: P<0.01 (siCocktail against siCntrl)). FIG. 21A is a graph showing the concentration of ALP in the serum of each mouse (N=5, *: P<0.05 (siCocktail against siCntrl). Further, FIG. 21B is a graph showing the concentration of T-BIL in the serum of each mouse (N=5, *: P<0.05 (siCocktail against siCntrl)).

As shown in FIGS. 20A to 21B, in the mouse treated with either SMO-siRNA or TGFβ1-siRNA alone, liver function was partially improved but not fully restored, whereas in the mouse treated with the combination, liver function was restored to a level close to that of a healthy state.

It should be noted that the sequences of SMO-siRNA and TGFβ1-siRNA used in this experiment (SEQ ID NOs: 1 to 4) are merely examples, and similar effects can be obtained with other sequences.

This application is entitled to and claims the benefit of Japanese Patent Application No. 2022-121626, filed on Jul. 29, 2022, the disclosure of which including the specification and drawings is incorporated herein by reference in its entirety.

### Industrial Applicability

A lipid nanoparticle according to the present invention can efficiently deliver a target substance such as RNA to a hepatic stellate cell, and thus the lipid nanoparticle is useful for medicine for treatment or prevention of diseases involving hepatic stellate cells, such as hepatic fibrosis. A pharmaceutical composition and a method according to the present invention are also useful for the prevention or treatment of hepatic fibrosis.

## Claims

1. A lipid nanoparticle for delivering a target substance to a hepatic stellate cell, the lipid nanoparticle comprising:
a pH-sensitive cationic lipid including a hydrophilic portion and two hydrophobic portions, wherein
an acid dissociation constant pKa of a lipid membrane constituting the lipid nanoparticle is 6.7 or more and less than 8.2.

2. The lipid nanoparticle according to claim 1, wherein the hydrophilic portion includes a 5-7 membered non-aromatic heterocyclic group bonded to a carbonyl group.

3. The lipid nanoparticle according to claim 2, wherein:
the two hydrophobic portions each independently includes an alkylene group having 4 to 12 carbon atoms, and an aliphatic group bonded to the alkylene group or a fatty acid ester group bonded to the alkylene group;
the aliphatic group is a saturated or unsaturated aliphatic group having 8 to 18 carbon atoms and having 0 to 2 unsaturated bonds; and
the fatty acid ester group is a linear or branched saturated or unsaturated fatty acid ester group having 10 to 24 carbon atoms and having 0 to 2 unsaturated bonds.

4. The lipid nanoparticle according to claim 1, wherein:
the hydrophilic portion includes, at the end thereof, an ionizable tertiary amino group; and
the two hydrophobic portions each independently include an alkylene group having 4 to 12 carbon atoms and a fatty acid ester group having 15 or more carbon atoms, the fatty acid ester group being bonded to the alkylene group.

5. The lipid nanoparticle according to claim 3, wherein:
the pH-sensitive cationic lipid is represented by a general formula (1) below:
(R¹)(R²)C(OH)-(CH₂)ₐ-(O-CO)-X¹ ... (1)
in the general formula (1), a represents an integer of 3 to 5, and R¹ and R² each independently represent a group represented by a general formula (2) or (3) below:
CH₃-(CH₂)_{q}-(CH=CH)ᵣ-(CH₂)ₛ-(CH=CH)ₜ-(CH₂)ᵤ-(CO-O)_{c}-(CH₂)ᵥ- ... (2)
in the general formula (2), q represents an integer of 1 to 9, r represents 0 or 1, s represents an integer of 1 to 3, t represents 0 or 1, u represents an integer of 1 to 8, c represents 0 or 1, and v represents an integer of 4 to 12, and
(R³)(R⁴)C-(CO-O)-(CH₂)ᵥ- ... (3)
in the general formula (3), R³ and R⁴ are each independently a C₃₋₁₁ alkyl group, and v is an integer of 4 to 12, and
X¹ represents the 5-7 membered non-aromatic heterocyclic group that is bonded to (O-CO)- via a carbon atom, and a hydrogen atom on a ring of the 5-7 membered non-aromatic heterocyclic group is optionally substituted with one or two C₁₋₄ alkyl groups or C₂₋₄ alkenyl groups.

6. The lipid nanoparticle according to claim 4, wherein:
the pH-sensitive cationic lipid is represented by a general formula (4) below:
(R⁵)(R⁶)C(OH)-(CH₂)ₐ-X² ... (4)
in the general formula (4), a represents an integer of 3 to 5, and R⁵ and R⁶ each independently represent a group represented by a general formula (5) below:
CH₃-(CH₂)_{w}-(CH=CH)ₓ-(CH₂)_{y}-(CO-O)-(CH₂)ᵥ- ... (5)
in the general formula (5), w represents an integer of 1 to 15, x represents 0 or 1, y represents an integer of 1 to 15, and v represents an integer of 4 to 12, and
X² represents an ionizable tertiary amino group.

7. The lipid nanoparticle according to claim 5, wherein:
the X¹ is a 1-pyrrolidinyl group, a 1-piperidinyl group, a 1-morpholinyl group, or a 1-piperazinyl group; and
a hydrogen atom of the 1-pyrrolidinyl group, the 1-piperidinyl group, the 1-morpholinyl group or the 1-piperazinyl group is optionally substituted with one C₁₋₄ alkyl group.

8. The lipid nanoparticle according to any one of claims 1 to 7, wherein the acid dissociation constant pKa of the lipid membrane constituting the lipid nanoparticle is in a range of 6.7 to 7.4.

9. The lipid nanoparticle according to any one of claims 1 to 8, wherein a number average particle size of the lipid nanoparticle measured by dynamic light scattering is 100 nm or less.

10. The lipid nanoparticle according to any one of claims 1 to 9, wherein a ratio of the pH-sensitive cationic lipid to total lipids constituting the lipid nanoparticle is 20 mol% or more.

11. The lipid nanoparticle according to claim 10, further comprising:
20 to 30 mol% of a phospholipid containing a phosphoethanolamine group with respect to the total lipids constituting the lipid nanoparticle;
20 to 30 mol% of cholesterol with respect to the total lipids constituting the lipid nanoparticle; and
3 to 5 mol% of a polyalkylene glycol modified lipid with respect to the total lipids constituting the lipid nanoparticle.

12. The lipid nanoparticle according to any one of claims 1 to 11, wherein the target substance is RNA.

13. The lipid nanoparticle according to claim 12, wherein:
the target substance is mRNA; and
a nitrogen/phosphate ratio of the lipid nanoparticle is in a range of 6 to 14.

14. The lipid nanoparticle according to claim 12, wherein:
the target substance is siRNA; and
a nitrogen/phosphate ratio of the lipid nanoparticle is in a range of 4 to 8.

15. The lipid nanoparticle according to any one of claims 1 to 14, wherein the lipid nanoparticle does not include a ligand for binding to the hepatic stellate cell.

16. A pharmaceutical composition for preventing or treating liver fibrosis, the pharmaceutical composition comprising:
a lipid nanoparticle having delivery selectivity to a hepatic stellate cell;
a first substance that is encapsulated in the lipid nanoparticle and suppresses an expression of a SMO gene or a SMO protein or inhibits the SMO protein; and
a second substance that is encapsulated in the lipid nanoparticle and suppresses an expression of a TGFβ1 gene or a TGFβ1 protein or inhibits the TGFβ1 protein.

17. The pharmaceutical composition according to claim 16, wherein
the lipid nanoparticle is the lipid nanoparticle according to any one of claim 1 to 15.

18. The pharmaceutical composition according to claim 16 or 17, wherein:
the first substance is siRNA, miRNA, shRNA, an antisense oligonucleotide, or a component of CRISPR-Cas each capable of suppressing the expression of the SMO gene; and
the second substance is siRNA, miRNA, shRNA, an antisense oligonucleotide, or a component of CRISPR-Cas each capable of suppressing the expression of the TGFβ1 gene.
